(19) Europäisches Patentamt
European Patent Office
Office européen des brevets

(11) EP 3 305 336 A1

(12) EUROPEAN PATENT APPLICATION
published in accordance with Art. 153(4) EPC

(43) Date of publication:
11.04.2018 Bulletin 2018/15

(21) Application number: 16799908.5

(22) Date of filing: 19.05.2016

(51) Int Cl.:
*A61L 27/00* (2006.01) *A61F 2/06* (2013.01)
*A61L 33/00* (2006.01) *D03D 3/02* (2006.01)
*D03D 15/00* (2006.01)

(86) International application number:
PCT/JP2016/064811

(87) International publication number:
WO 2016/190202 (01.12.2016 Gazette 2016/48)

(84) Designated Contracting States:
AL AT BE BG CH CY CZ DE DK EE ES FI FR GB GR HR HU IE IS IT LI LT LU LV MC MK MT NL NO PL PT RO RS SE SI SK SM TR
Designated Extension States:
BA ME
Designated Validation States:
MA MD

(30) Priority: 27.05.2015 JP 2015108022

(71) Applicant: Toray Industries, Inc.
Tokyo 103-8666 (JP)

(72) Inventors:
- TSUCHIKURA, Hiroshi
Otsu-shi
Shiga 520-2141 (JP)
- KANEKO, Takayuki
Nagoya-shi
Aichi 455-8502 (JP)
- YAMADA, Satoshi
Otsu-shi
Shiga 520-2141 (JP)
- TANAHASHI, Kazuhiro
Otsu-shi
Shiga 520-8558 (JP)
- KADOWAKI, Koji
Otsu-shi
Shiga 520-8558 (JP)
- KUWABARA, Atsushi
Otsu-shi
Shiga 520-2141 (JP)

(74) Representative: Kador & Partner
Corneliusstraße 15
80469 München (DE)

# (54) TUBULAR WOVEN FABRIC STRUCTURE

(57) The present invention provides a multi-layer tubular woven construct useful as a hose for transporting a fluid or a powder or for protecting linear bodies such as wires, cables and conduits, as a tubular filter, or as a base material of a vascular prosthesis. In particular, provided are a tubular woven construct in a tubular configuration woven by interlacing warp and weft yarns, the warp yarn containing at least in part an elastic fiber yarn having a filament fineness of 1.0 dtex or more, the weft yarn containing at least in part a microfiber yarn having a filament fineness of less than 1.0 dtex; and a vascular prosthesis using the tubular woven construct as a base material.

EP 3 305 336 A1

**Description**

TECHNICAL FIELD

The present invention relates to a multi-layer tubular woven construct. In particular, the present invention relates to a multi-layer tubular woven construct useful as a hose for transporting a fluid or a powder or for protecting linear bodies such as wires, cables and conduits, as a tubular filter, or as a base material of a vascular prosthesis.

BACKGROUND ART

Tubular fiber constructs are used for various industrial applications, such as hoses, reinforcements, protective materials and vascular prostheses. Depending on the usage, tubular fiber constructs are bent, wound in a spiral shape, or disposed in a meandering manner to fit a space. Accordingly, in order to prevent flattening or twisting of tubular fiber constructs in various usages, a high kink resistance (flexibility) is imparted to tubular fiber constructs, and various methods for imparting a high kink resistance have been proposed.

Various tubular constructs have been proposed, including, for example, a fabric sleeve for bundling and protecting elongated articles, such as wires, cables, hoses and conduits, said sleeve comprised of warp and fill ends having an open construction, said sleeve having a substantially circular cross-sectional configuration (Patent Literature 1); and a tubular article usable as a watertight seal or a packing member of a shield machine, the tubular article being composed of a tubular woven fabric having an internal surface provided with an airtight seal made of, for example, a rubber or a resin (Patent Literature 2). Tubular constructs are also used as vascular prostheses, which are medical devices used to replace diseased blood vessels as in arteriosclerosis or other diseases or to create a bypass or a shunt. Conventional vascular prostheses can be roughly classified into four types based on their materials: 1) vascular prostheses made of a textile, 2) vascular prostheses made of polytetrafluoroethylene, 3) vascular prostheses made of a biomaterial, and 4) vascular prostheses made of a synthetic macromolecular material. Of these vascular prostheses, vascular prostheses made of a woven, knitted, or non-woven textile made from fibers have high flexibility, but have the drawback of easily causing leakage of blood from the voids between the fibers due to the blood pressure applied to the vascular prostheses in actual use. Of these textile vascular prostheses, knitted vascular prostheses are not preferred because, although they are produced by a simple production process and have flexibility, they have poor shape-retaining ability and often have a porous structure, which tends to cause leakage of blood from the voids between the fibers. Non-woven vascular prostheses are also not preferred because they have an uneven structure and poor shape-retaining ability.

Woven vascular prostheses have an advantage over knitted vascular prostheses in that the amount of leakage of blood can be minimized by reducing the size of the voids between the fibers . Due to this advantage, woven vascular prostheses are used for vascular surgery such as aorta surgery, which is in high demand. Leakage of blood can be minimized generally by reducing the size and number of the voids between the fibers, but in such a method, the resulting vascular prosthesis has a high fiber density and is thus rigid. Use of such a rigid vascular prosthesis often renders surgery difficult because the cut ends of a native blood vessel from which a diseased portion has been removed, i.e., the ends of a native blood vessel to be connected with the vascular prosthesis, are also rigid due to arteriosclerosis or other diseases.

As an attempt to increase the flexibility of a textile vascular prosthesis, there has been proposed a vascular prosthesis using highly stretchable elastic fibers (Patent Literature 3). However, the vascular prosthesis has the drawbacks of having poor shape-retaining ability due to its structure made only with the elastic fibers and of easily causing leakage of blood from the voids between the fibers due to the large diameter of the fibers.

Based on these drawbacks, another method has been proposed in which leakage of blood from a textile vascular prosthesis used in vascular surgery is prevented not only by reducing the size and number of the voids between the fibers but by attaching a bioabsorbable gel, such as collagen and gelatin, to the vascular prosthesis to fill the voids (Patent Literature 4).

Another proposed method is the so-called preclotting, in which a textile vascular prosthesis is brought into contact with autologous blood immediately before implantation to allow formation of thrombi, thereby filling the voids between the fibers and preventing leakage of blood (Patent Literature 5 and 6) .

Blood vessels in a living body have an intima on the luminal surface, and vascular endothelial cells in blood vessels inhibit thrombus formation. Conventional vascular prostheses have, however, a low cellular affinity, which delays the settlement of vascular endothelial cells. Due to this, a long period of time is required for the settlement of vascular endothelial cells and the formation of the intima. Accordingly, vascular prostheses are required to exhibit not only antithrombogenicity immediately after implantation but also cellular affinity over time.

Cellular affinity of a textile vascular prosthesis can be increased by providing a fiber structure that promotes cell growth and infiltration. Examples of such a method include the optimization of the diameter of fibers, and raising of fibers, napping, and/or formation of looped fibers (Patent Literature 7 to 10).

A tubular woven fabric implanted as a vascular prosthesis is typically recognized as foreign by the body. In particular, blood coagulation reaction proceeds on the surface in contact with blood, i.e., the inner surface of the vascular prosthesis, leading to the formation of thrombi. In order to prevent this, antithrombogenicity is required.

Antithrombogenicity of a medical material can conventionally be enhanced by attaching heparin or a heparin derivative to a surface of the material. However, heparin or a heparin derivative cannot be directly attached to particular types of vascular prostheses, such as a vascular prosthesis made of a medical textile material made of polyester fibers etc. and a vascular prosthesis made of a medical material made of porous expanded polytetrafluoroethylene (hereinafter referred to as "ePTFE"). To overcome this problem, there have been proposed methods to covalently bind heparin or a heparin derivative to a modified surface of a medical material (Patent Literature 11 to 13), and methods to ionically bind heparin or a heparin derivative to a surface of a material (Patent Literature 14 to 17) .

Several other methods for imparting antithrombogenicity to a textile vascular prosthesis have also been proposed, including a method in which heparin or a heparin derivative is added to a bioabsorbable gel used for prevention of leakage of blood, such as collagen and gelatin, and the heparin-containing gel is attached to a surface of a material (Patent Literature 6 and 18); and a method in which a segmented polyurethane dissolved in an organic solvent is impregnated into a material, thereby attaching the polyurethane on a surface of the material (Patent Literature 19).

Methods for enhancing antithrombogenicity of a medical material by using an antithrombogenic compound other than heparin or a heparin derivative have also been proposed, including a method in which a particular compound is attached to a surface of a medical material. The compound is exemplified by a compound that inhibits blood coagulation factors involved in the blood coagulation reaction (e.g., platelets, which are involved in the primary hemostasis) , a compound that inhibits thrombin, which is involved in the thrombus formation (Patent Literature 20 to 22).

Blood vessels in a living body have an intima on the luminal surface, and vascular endothelial cells in blood vessels inhibit thrombus formation. Conventional vascular prostheses have, however, a low cellular affinity, which delays the settlement of vascular endothelial cells. Due to this, a long period of time is required for the settlement of vascular endothelial cells and the formation of the intima. Accordingly, vascular prostheses are required to exhibit not only antithrombogenicity immediately after implantation but also cellular affinity over time.

CITATION LIST

PATENT LITERATURE

Patent Literature 1: JP Patent No. 2718571
Patent Literature 2: JP 2003-329146 A
Patent Literature 3: JP H08-80342 A
Patent Literature 4: JP Patent No. 3799626
Patent Literature 5: JP H05-48132 B
Patent Literature 6: JP H05-88611 B
Patent Literature 7: JP S61-4546 B
Patent Literature 8: JP S61-58190 B
Patent Literature 9: JP S63-52898 B
Patent Literature 10: JP H05-28143 B
Patent Literature 11: JP 2009-545333 A
Patent Literature 12: JP Patent No. 4152075
Patent Literature 13: JP Patent No. 3497612
Patent Literature 14: JP S60-41947 B
Patent Literature 15: JP S60-47287 B
Patent Literature 16: JP Patent No. 4273965
Patent Literature 17: JP H10-151192 A
Patent Literature 18: JP H08-24686 B
Patent Literature 19: JP H07-265338 A
Patent Literature 20: JP Patent No. 4461217
Patent Literature 21: WO 08/032758
Patent Literature 22: WO 12/176861

SUMMARY OF INVENTION

TECHNICAL PROBLEM

The tubular fabric sleeve of Patent Literature 1 is discontinuous in the circumferential direction and thus has a narrow gap or opening extending in the longitudinal direction along the discontinuous part. The narrow gap or opening may cause leakage of a fluid or a powder during transportation, or may allow penetration of linear bodies, such as wires, cables, hoses and conduits. The literature also describes a configuration in which the edges of the longitudinal slit are overlapped to close the opening. The overlapped part forms a raised seam on the inner surface. The raised part may affect feed pressure for transporting a fluid or a powder. In addition, linear bodies, such as wires, cables, hoses and conduits, may be caught by the uneven inner surface.

The tubular article of Patent Literature 2 requires a watertight means on the inner surface in order to prevent leakage of a fluid or a powder during transportation.

A tubular construct used as a vascular prosthesis also requires a means to prevent leakage of blood. In particular cases where the method disclosed in Patent Literature 3 is applied to a textile vascular prosthesis, the surface of the fibers needs to be coated with, for example, collagen or gelatin containing heparin or a heparin derivative.

A vascular prosthesis with a highly porous structure, i.e., a woven structure with high water permeability, is disclosed in Patent Literature 5 and 6. In Patent Literature 5, the highly porous structure promotes the settlement of vascular endothelial cells on the inner surface of the vascular prosthesis, thereby promoting the formation of the intima. In Patent Literature 6, the highly porous structure minimizes the amount of foreign matter in contact with the native tissue, thereby increasing biocompatibility. However, these vascular prostheses essentially require preclotting, and due to this procedure, thrombi are formed and eventually destroy the fine structure formed by thin fibers with a small diameter and the voids between the fibers, leading to a decrease in cellular affinity. Another problem may occur when an anticoagulant (e.g., heparin or argatroban) is used in vascular surgery. An anticoagulant is commonly used in vascular surgery for prevention of blood coagulation. However, due to the use of an anticoagulant, thrombus formation is less likely to occur, and as a result, preclotting may be insufficient to fill the voids between the fibers. In some cases, thrombi formed by preclotting are lysed by the action of the fibrinolytic system in the blood after surgery, which may lead to leakage of blood.

Patent Literature 7 to 10 discloses that a vascular prosthesis containing fibers of 0.5 denier or less, i.e., 0.56 dtex or less in at least part of the inner surface has a certain degree of cellular affinity, but the cellular affinity can be further enhanced by raising fibers, napping, and/or forming looped fibers. However, this method is disadvantageous in that an additional procedure is required to raise fibers, nap, and/or form looped fibers, and that the additional procedure produces waste fibers. A further disadvantage is that the orientation of fibers in the warp and weft is largely disturbed, which hinders the settlement of vascular endothelial cells and may result in a decrease in cellular affinity.

Patent Literature 11 to 13 discloses a method in which heparin or a heparin derivative is covalently or ionically bound to a surface modifier, which is then attached to a surface of a medical material. However, none of the literature mentions a textile vascular prosthesis, and the base material used in the literature is not a tubular woven fabric with elasticity and flexibility.

Patent Literature 18 and 19 discloses a method in which a bioabsorbable gel containing heparin or a heparin derivative or an organic solvent containing an antithrombogenic material is physically attached to a surface of a medical material. However, none of the literature specifies the fiber diameter that is advantageous for promoting cell growth etc. on a textile vascular prosthesis, and the base material used in the literature is not a tubular woven fabric with elasticity and flexibility.

Patent Literature 20 to 22 discloses a method in which a compound having antithrombogenicity is immobilized on a surface of a medical material. The compound may be a combination of two compounds each having both anti-platelet adhesion activity and anti-thrombin activity, or a single compound prepared by combining a compound with anti-platelet adhesion activity and a compound with anti-thrombin activity into one molecule. However, the base material used in the literature is also not a tubular woven fabric with elasticity and flexibility.

As described above, there has not been available a base material that can be used to provide a textile vascular prosthesis made of a tubular woven construct that causes minimal leakage of blood and has both antithrombogenicity and cellular affinity. Currently available vascular prostheses with a small luminal diameter of below 6 mm are prone to thrombus formation due to a small flow of blood. Even a small thrombus may be a size equivalent to the luminal diameter of the vascular prosthesis and may easily inhibit the blood flow. Current vascular prostheses with a small luminal diameter cannot achieve good performance in the long run, and therefore cannot be applied to clinical practice.

The present invention was made to solve the above problems in the conventional art. An object of the present invention is therefore to provide a multi-layer tubular woven construct that has excellent mechanical strength, excellent mechanical properties such as elasticity and flexibility, and excellent physical properties such as kink resistance, and is capable of transporting a fluid or a powder without causing problems, and is suitable as a hose for protecting linear bodies such as wires, cables and hoses, as a tubular filter, or as a base material of a vascular prosthesis. The tubular

woven construct of the present invention, when used as a base material of a vascular prosthesis, causes minimal leakage of blood and has both antithrombogenicity and cellular affinity as well as the above properties, thereby serving as a textile vascular prosthesis.

SOLUTION TO PROBLEM

The inventors conducted extensive research to solve the above problems, and as a result, completed the present invention as described in the following (1) to (17).

(1) A tubular woven construct in a tubular configuration woven by interlacing warp and weft yarns, the warp yarn containing at least in part an elastic fiber yarn having a filament fineness of 1.0 dtex or more, the weft yarn containing at least in part a microfiber yarn having a filament fineness of less than 1.0 dtex.
(2) The tubular woven construct according to the above (1), which satisfies the following formula: Cfa < Cfb, wherein Cfa is a warp cover factor and Cfb is a weft cover factor.
(3) The tubular woven construct according to the above (1) or (2), which has an elongation of 4% or more in the warp direction per mm in width of the tubular woven construct under a load of 3.3 N, and has an elongation at break of 50% or less.
(4) The tubular woven construct according to any one of the above (1) to (3), wherein the elastic fiber yarn having a filament fineness of 1.0 dtex or more contains composite cross-section fiber filaments formed of two types of polymers having different thermal shrinkage properties.
(5) The tubular woven construct according to the above (4), wherein the two types of polymers having different thermal shrinkage properties are polyethylene terephthalate and polytrimethylene terephthalate.
(6) The tubular woven construct according to any one of the above (1) to (5), which contains two or more layers.
(7) The tubular woven construct according to the above (6), wherein a layer other than an innermost layer comprises a weft yarn containing at least in part a monofilament yarn having a thickness of 20 μm or more.
(8) The tubular woven construct according to any one of the above (1) to (7), whose inner surface has a water permeability of 500 mL/min-120 mmHg (16 kPa) $\cdot cm^2$ or less.
(9) A vascular prosthesis containing the tubular woven construct according to any one of the above (1) to (8) as a base material.
(10) The vascular prosthesis according to the above (9), which has an antithrombogenic material layer formed by binding of an antithrombogenic material to an inner surface of the tubular woven construct to be in contact with blood, wherein the antithrombogenic material layer has a thickness of 1 to 600 nm.
(11) The vascular prosthesis according to the above (10), wherein the antithrombogenic material contains a sulfur-containing anionic compound having anticoagulant activity.
(12) The vascular prosthesis according to the above (10) or (11), whose inner surface, when subjected to X-ray photoelectron spectroscopy (XPS), shows an abundance ratio of sulfur atoms of 3.0 to 6.0 atomic percent relative to all the atoms on the inner surface.
(13) The vascular prosthesis according to any one of the above (10) to (12), whose inner surface, when subjected to X-ray photoelectron spectroscopy (XPS), shows an abundance ratio of nitrogen atoms of 6.0 to 12.0 atomic percent relative to all the atoms on the inner surface.
(14) The vascular prosthesis according to any one of the above (10) to (13), wherein the antithrombogenic material contains a cationic polymer containing, as a constituent monomer, a compound selected from the group consisting of alkyleneimines, vinyl amines, allylamine, lysine, protamines, and diallyl dimethyl ammonium chloride, and wherein the cationic polymer is covalently bound to warp and weft yarns that form the tubular woven construct.
(15) The vascular prosthesis according to the above (10), wherein the antithrombogenic material is a compound containing three types of skeletal structures, wherein the three types of skeletal structures are a hydrophilic polymer skeleton, a 4-(aminomethyl)benzenecarboxyimidamide or benzamidine skeleton, and a methoxy benzene sulfonamide skeleton, wherein the hydrophilic polymer skeleton contains, as a constituent monomer, a compound selected from the group consisting of ethylene glycol, propylene glycol, vinylpyrrolidone, vinyl alcohol, vinyl caprolactam, vinyl acetate, styrene, methyl methacrylate, hydroxyethyl methacrylate, and siloxane, and wherein the compound containing the three types of skeletal structures is covalently bound to warp and weft yarns that form the tubular woven construct.
(16) The vascular prosthesis according to the above (15), wherein the compound containing the three types of skeletal structures is a compound represented by any of the following general formulae (I) to (IV):

(I)

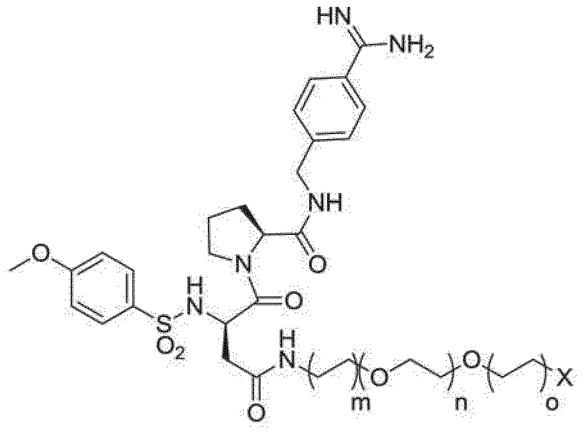

,

(II)

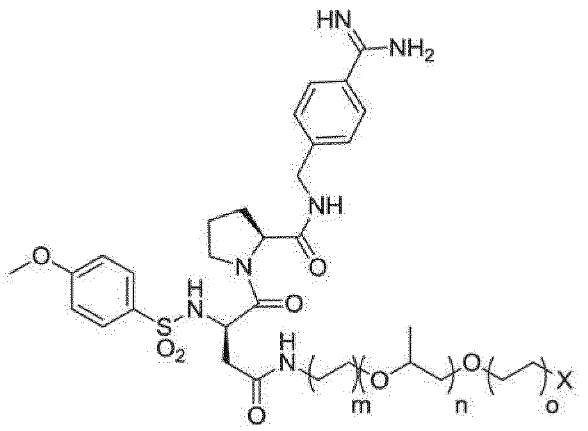

,

(III)

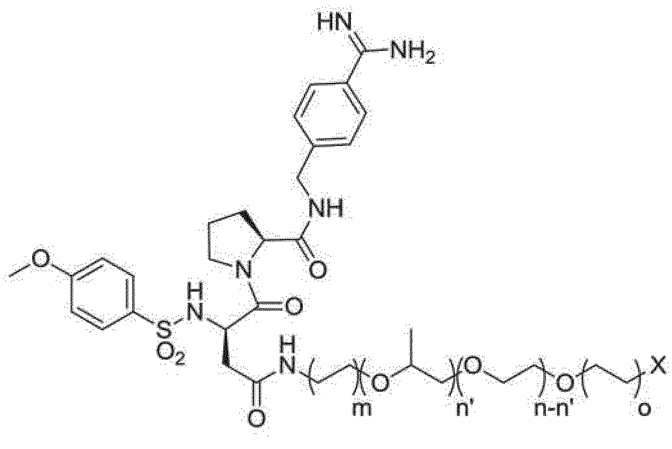

,

and

(IV)

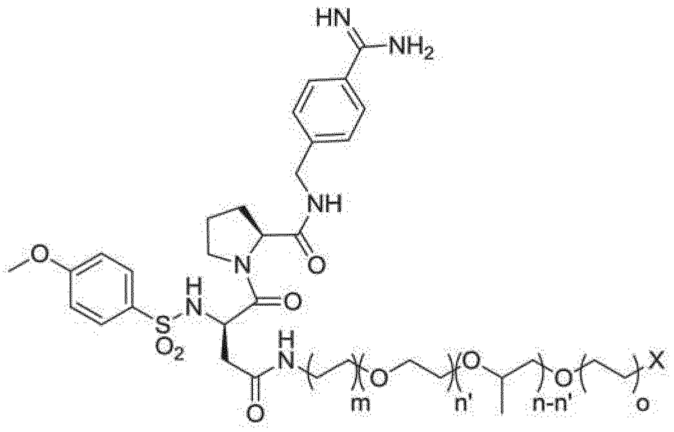

,

wherein m and o each represent an integer of 0 to 4; n represents an integer of 3 to 1000, and n' represents an integer of 3 to 1000, with the proviso that n and n' satisfy the formula: n ≥ n' ; and X represents a functional group selected from the group consisting of hydroxyl, thiol, amino, carboxyl, aldehyde, isocyanate, and thioisocyanate groups.

(17) The vascular prosthesis according to any one of the above (10) to (16), wherein the antithrombogenic material contains an anionic polymer containing, as a constituent monomer, a compound selected from the group consisting of acrylic acid, methacrylic acid, α-glutamic acid, γ-glutamic acid and aspartic acid; or an anionic compound selected from the group consisting of oxalic acid, malonic acid, succinic acid, fumaric acid, glutaric acid, adipic acid, pimelic acid, suberic acid, azelaic acid, sebacic acid, malic acid, tartaric acid, and citric acid.

ADVANTAGEOUS EFFECTS OF INVENTION

According to a first aspect of the present invention as specified in claim 1, the tubular woven construct of the present invention has excellent mechanical strength, excellent mechanical properties such as elasticity and flexibility, and excellent physical properties such as kink resistance and is capable of transporting a fluid or a powder without causing problems. The tubular woven construct is therefore suitable as a hose for protecting linear bodies, as a hose for transporting a fluid, as a tubular filter, as a packing, or as a base material of a vascular prosthesis. The tubular woven construct of the present invention, when used as a base material of a vascular prosthesis, causes minimal leakage of blood and has both antithrombogenicity and cellular affinity as well as the above properties. The tubular woven construct may have an antithrombogenic material layer of an appropriate thickness as specified in claim 10, and such a tubular woven construct has the optimum antithrombogenicity.

DESCRIPTION OF EMBODIMENTS

The tubular woven construct of the present invention is a woven fabric in a tubular configuration woven by interlacing warp and weft yarns, wherein an elastic fiber yarn having a filament fineness of 1.0 dtex or more is used in at least part of the warp, and a microfiber yarn having a filament fineness of less than 1.0 dtex is used in at least part of the weft.

Unless otherwise specified, the terms used herein have the definitions described below.

The term "tubular woven fabric" as used herein means a woven fabric in a tubular configuration woven by interlacing warp and weft yarns. In the tubular woven construct of the present invention, the warp yarn contains at least in part an elastic fiber yarn having a filament fineness of 1.0 dtex or more, and the weft yarn contains at least in part a microfiber yarn having a filament fineness of less than 1.0 dtex.

The term "filament fineness" means a value obtained by dividing the total fineness of a yarn by the number of filaments in the yarn. The total fineness is determined as a mass-corrected fineness in accordance with method A in JIS L 1013 (2010) 8.3.1 under a predetermined load of 0.045 cN/dtex.

Warp yarn contains at least in part an elastic fiber yarn having a filament fineness of 1.0 dtex or more

The warp yarn containing at least in part an elastic fiber yarn having a filament fineness of 1.0 dtex or more

imparts not only a high mechanical strength but also elasticity and flexibility to the tubular woven construct, thereby achieving high physical properties such as a high kink resistance. If the warp yarn contains no elastic fiber yarn having a filament fineness of 1.0 dtex or more, not only the mechanical strength but also the elasticity and flexibility of the tubular woven construct tend to be low. The warp yarn containing at least in part an elastic fiber yarn having a filament fineness of 2.0 dtex or more is preferred because the strength, elasticity and flexibility are maintained at an excellent level even in cases of long-term use where the polymers that form the fibers in the tubular woven construct may undergo hydrolysis causing strength deterioration, or may undergo creep deformation causing a reduction in the elasticity and/or flexibility. The filament fineness of the elastic fiber yarn is preferably 5.0 dtex or less, more preferably 3.0 dtex or less, for achieving adequate flexibility.

A multifilament yarn containing a plurality of filaments is preferred as the warp yarn for achieving adequate flexibility. The number of the filaments contained in the multifilament yarn is not particularly limited, but is preferably 5 or more, more preferably 15 or more.

The total fineness of the elastic fiber yarn is preferably from 5 dtex to 200 dtex.

The elastic fiber yarn having a total fineness of 5 dtex or more is not excessively thin and achieves sufficient strength and elasticity. The elastic fiber yarn having a total fineness of 200 dtex or less is not excessively thick and imparts excellent flexibility to the tubular woven construct.

The elastic fiber yarn used in the present invention is a fiber yarn with elasticity (having a high elongation and a high recovery percentage of elongation) . The elastic fiber yarn is not limited to a particularly type as long as it has a recovery percentage of elongation of 30% or more when the yarn is stretched by 20% (stretch rate of 20%) by applying a load, and the recovery percentage of elongation is preferably 40% or more, more preferably 50% or more. The recovery percentage of elongation when the yarn is stretched by 10% (stretch rate of 10%) is 50% or more, preferably 60% or more, more preferably 80%. However, when the recovery percentage of elongation is excessively high, the repulsive force against deformation may be excessively high and thus the tubular woven construct may be excessively rigid. Because of this reason, the recovery percentage of elongation when the yarn is stretched by 20% (stretch rate of 20%) is preferably up to 90%.

Specific examples of a preferred elastic fiber yarn include a spandex fiber yarn, and a composite cross-section fiber yarn formed of two types of polymers having different thermal shrinkage properties.

The spandex fiber yarn may be a common elastic yarn called spandex, such as a polyurethane fiber yarn etc. The spandex fiber yarn is preferably a covered yarn having a sheath made of synthetic fibers, such as nylon or polyester fibers, wound around a spandex core.

The composite cross-section fiber yarn formed of two types of polymers having different thermal shrinkage properties is preferably a composite fiber yarn to which latent crimpability has been imparted by use of two types of polymers having different thermal shrinkage properties. The composite cross-section fiber yarn is preferably in the form of a multifilament yarn composed of a plurality of filaments. The composite cross-section fiber yarn is preferably a composite cross-section fiber yarn that has latent crimpability due to having a composite structure in which two types of polymer components having different thermal shrinkage properties are arranged in a side-by-side configuration or an eccentric core-sheath configuration along the longitudinal direction of the yarn. The composite cross-section fiber yarn with this configuration may be in the form of a multifilament that has been subjected to false twisting or heat treatment to form coil-shaped crimps, which impart high elasticity.

The elastic fiber yarn used in the present invention is preferably a composite cross-section fiber yarn formed of two types of polymers having different thermal shrinkage properties. More preferably, the composite cross-section fiber yarn is a multifilament yarn of composite cross-section fibers in which two types of polymer components having different thermal shrinkage properties are arranged in a side-by-side configuration or an eccentric core-sheath configuration along the longitudinal direction. Still more preferably, the composite cross-section fiber yarn is a multifilament yarn of the composite fibers with the configuration as described above to which high elasticity has been imparted by coil-shaped crimps created by false twisting or heat treatment.

The warp threads made of the elastic fiber yarn preferably account for at least 50% or more of the total number of the warp threads in the tubular woven construct of the present invention. The warp threads made of the elastic fiber yarn more preferably account for 80% or more, most preferably 100%, of the total number of the warp threads.

<u>Weft yarn contains at least in part a microfiber yarn having a filament fineness of less than 1.0 dtex</u>

The weft yarn containing at least in part a microfiber yarn having a filament fineness of less than 1.0 dtex imparts not only high flexibility but also a low water permeability to the tubular woven construct due to the fine filaments and the small size of voids between the fibers. The term "microfiber yarn" as used herein means fibers having a filament fineness of less than 1.0 dtex. The microfiber yarn is preferably in the form of a multifilament yarn.

When a multifilament yarn having a filament fineness of 1.0 dtex or more is used alone as the weft yarn, a low water permeability can be achieved if the weave density is high, but the resulting tubular woven construct is excessively

rigid and has low flexibility and low elasticity. An excessively rigid woven construct may cause kinking and may have an uneven surface on the inner layer. Therefore, sole use of such a multifilament yarn having a filament fineness of 1.0 dtex or more is not preferred.

The microfiber yarn in the present invention may be a single type or a combination of different types of microfiber yarns with different filament finenesses and different total finenesses.

The microfiber yarn that may be used is the so-called direct spun yarn obtained by the so-called direct melt spinning; or a splittable yarn that can be made into ultra-fine fibers by splitting splittable filaments having a composite cross section.

The splittable yarn may be one that can be made into ultra-fine fibers by chemical or physical means. The ultra-fining process may be performed after the tubular woven fabric is formed, or alternatively, before the tubular woven fabric is formed, but preferably the ultra-fining process is performed after the tubular woven fabric is formed due to the reasons described later. The ultra-fining process by chemical or physical means may be done by, for example, removing one of the components in a composite fiber yarn or by splitting a composite fiber yarn into its respective segments, thereby giving fibrils or ultra-fine fibers, as described in U.S. Pat. No. 3531368 and U.S. Pat. No. 3350488. By the process, fibers of normal thickness at the time of the formation of the multi-layer tubular woven fabric can be made into ultra-fine fibers at a later process. Consequently, troubles that may occur during various processing, for example, breakage of a yarn and formation of lint during the weaving process or during various yarn processing before weaving, are minimized.

The microfiber yarn used as the weft yarn of the tubular woven construct of the present invention may be made of various types of organic fibers, but polyester fibers are preferred in terms of water absorbability and degradation resistance. Examples of the polyester fibers include polyethylene terephthalate fibers, polybutylene terephthalate fibers, etc. The polyester fibers may be copolymerized polyester fibers produced by copolymerizing polyethylene terephthalate, polybutylene terephthalate, or the like with an acid component, for example, isophthalic acid, sodium 5-sulfoisophthalate, or an aliphatic dicarboxylic acid such as adipic acid. The fibers contained in the multifilament yarn or the fibers contained in the warp and weft yarns may be a single type or an appropriate combination of different types of fibers.

Prevention of blood leakage and achievement of cellular affinity with use of microfibers

The tubular woven construct of the present invention containing a microfiber yarn having a filament fineness of less than 1.0 dtex in the weft has a small number of voids between the fibers, and therefore, when used as a vascular prosthesis, is less likely to cause leakage of blood. In addition, the inner layer has a very large number of scaffolds suitable for the adhesion of vascular endothelial cells. As a result, vascular endothelial cells are well settled on the structural fibers of the inner layer of the vascular prosthesis, and the cells well adhere to the inner layer of the vascular prosthesis . Therefore, the tubular woven construct can serve as a vascular prosthesis with high biocompatibility. The microfiber yarn used in the vascular prosthesis has a filament fineness of less than 1.0 dtex, preferably 0.50 dtex or less. The microfiber yarn having a filament fineness of 0.008 dtex or more is preferred because the cells well adhere to the surface. The total fineness of the microfiber yarn is preferably from 5 dtex to 200 dtex.

A microfiber yarn having a total fineness of less than 5 dtex is too thin to obtain enough strength. On the other hand, a microfiber yarn having a total fineness of more than 200 dtex is excessively thick and the resulting tubular woven construct may have low flexibility, which may lead to a low kink resistance.

Preclotting

Blood pressure is maintained at a certain high level in a living body, and due to this, when a woven construct is used as a vascular prosthesis, the leakage of blood through the voids between the fibers is difficult to avoid. Accordingly, before use of a textile vascular prosthesis in vascular surgery, the so-called preclotting is often performed. Preclotting is a pre-implantation procedure in which a vascular prosthesis is brought into contact with blood to artificially form thrombi, which temporally clog the voids between the fibers.

In current surgical operations, however, heparin is often used to prevent the coagulation of blood. Consequently, it is often the case that clogging by preclotting becomes insufficient, which leads to a risk that the leakage of blood may occur and may result in massive bleeding after surgery. Another risk is that, after surgery, fibrin produced by preclotting may begin to be dissolved by fibrinolysis as a natural phenomenon and the coagulated blood tissue may be easily broken.

Accordingly, in cases where a medical textile material is used in aortic and cardiac surgery using a large amount of heparin, a biodegradable substance such as collagen and gelatin is applied to the textile material to prevent the leakage of blood by not allowing the blood to infiltrate the textile material. This technique is utilized for the so-called coated vascular prosthesis and the so-called coated prosthetic patch, and they are already commercially available. However, since many of the substances (such as collagen and gelatin) used to create clogging of the voids in the textile material for the preparation of a coated vascular prosthesis or a coated prosthetic patch are naturally occurring substances, the stabilization of the quality of the substances is very difficult. Therefore these substances are not suitable for industrial application.

Before describing how the vascular prosthesis of the present invention effectively promotes the settlement of vascular endothelial cells, an assumed mechanism of prevention of the leakage of blood by microfibers will be described below.

Blood coagulation starts from fibrin formation and platelet aggregation. Fibrin formation is affected by heparin administration or fibrinolysis as described above, whereas platelet aggregation is less affected by them. Based on this, the inventors attempted to utilize the platelet aggregation pathway and, to this end, focused on the diameter of the structural fibers of the vascular prosthesis.

When platelets come into contact with a foreign body other than the surface of vascular endothelial cells, the platelets adhere to the surface of the foreign body. When the stimulus from the foreign body is large, platelets rupture and release their internal granules into surroundings, and the platelet debris adheres to the site where they rupture. The spread granules adhere to other platelets and stimulate them to rupture and release their granules just like a chain reaction. The ruptured platelets leave the debris. The debris and granules gather one after another and aggregate to form a thrombus. Since the size of platelets is about 1 to 2 $\mu$m, a microfiber yarn having a filament fineness of less than 1.0 dtex will easily capture platelets. In this manner, a thrombus grown by the above mechanism adheres to the ultra-fine microfibers. Once platelet aggregation is started, fibrin formation is spontaneously induced. Consequently, the leakage of blood is effectively prevented.

Cover factor

The tubular woven construct of the present invention preferably satisfies the following formula: Cfa < Cfb, wherein Cfa is a warp cover factor and Cfb is a weft cover factor. The cover factor indicates the degree of the density of voids between the fibers (packing density) . A smaller cover factor means the presence of a larger size of voids between the fibers. Accordingly, the tubular woven construct of the present invention that satisfies the formula Cfa (warp cover factor) < Cfb (weft cover factor) is preferred because microfibers occupy a large surface area, and thereby water permeability and leakage of blood are maintained at a minor level.

In cases where the tubular woven construct of the present invention has a multilayer structure containing two layers or more and where the tubular woven construct is used as a transfer tube for a fluid or a powder, as a vascular prosthesis, or as a protective material for wires or electric wires, it is especially advantageous that the cover factor of the innermost layer to be in contact with the contents of the tubular construct satisfies the above relation Cfa < Cfb. As long as the cover factor of the innermost layer satisfies the relation Cfa < Cfb, even when the woven structure of a layer other than the innermost layer does not satisfy the relation Cfa < Cfb, the tubular woven construct can be used as appropriate.

The cover factor is a value measured by the method described later.

The warp cover factor is preferably from 500 to 2000, more preferably from 1000 to 2000. The weft cover factor is preferably from 1000 to 2000. The sum total of the warp and weft cover factors (Cfa + Cfb) indicates the degree of the density of the whole tubular woven construct. The total of the cover factors (Cfa + Cfb) is preferably from 1500 to 4000, more preferably from 1800 to 3000. When the total of the cover factors (Cfa + Cfb) is 1500 or more, the voids present in the woven structure are small, which reduces the concern of leakage of a powder or a liquid. When the total of the cover factors (Cfa + Cfb) is 4000 or less, a high density and a high flexibility are achieved.

Tubular woven construct has an elongation of 4% or more in the warp direction per mm in width of the tubular woven construct under a load of 3.3 N and an elongation at break of 50% or less

To achieve adequate stretchiness, the elongation in the warp direction per mm in width of the tubular woven construct under a load of 3.3 N is preferably 4% or more, more preferably 4.5% or more. The elongation in the warp direction is preferably up to 15%, more preferably up to 10%. The elongation in the warp direction per mm in width of the tubular woven construct under a load of 3.3 N is determined by the method described later.

The tubular woven construct having an elongation at break of 50% or less is preferred because it has adequate dimensional stability and stretchiness as well as excellent flexibility. The elongation at break is more preferably 40% or less. The tubular woven construct having an elongation at break of 10% or more is preferred for achieving adequate flexibility. The elongation at break is more preferably 20% or more.

The tubular woven construct of the present invention has a large elongation under a small load and can thus easily elongate in response to a small external force applied thereto, while having a small elongation at break, and is thus excellent in dimensional stability. Such characteristics are favorable especially when the tubular woven construct is used as a base material of a vascular prosthesis. The vascular prosthesis preferably has stretchability so as to imitate autologous blood vessels (blood vessels in a living body), which constrict and dilate in response to changes in the blood

pressure and thereby control fluctuations in the blood pressure and in the blood flow.

The tubular woven construct of the present invention containing the elastic fiber yarn in the warp has excellent stretchability. The above preferred ranges for the elongation and the elongation at break of the tubular woven construct can be achieved by appropriately adjusting the elongation, the recovery percentage of elongation, the warp cover factor etc. of the elastic multifilament yarn. Excellent stretchability as described above can be easily exhibited when the elastic fiber yarn having a filament fineness of 1.0 dtex or more in the warp is a composite cross-section fiber yarn formed of two types of polymers having different thermal shrinkage properties.

Composite cross-section fiber yarn formed of two types of polymers having different thermal shrinkage properties

The polymers used to form the composite cross-section fiber yarn formed of two types of polymers having different thermal shrinkage properties are preferably two different types of polyesters having different thermal shrinkage properties. Preferred combinations of the polyesters include a combination of polyesters (e.g., polytrimethylene terephthalates etc.) with different viscosities, and a combination of a polytrimethylene terephthalate and another type of polyester (e.g., a polyethylene terephthalate, a polybutylene terephthalate, etc.). Especially preferred is a combination of a polyethylene terephthalate (PET)-based polyester and a polytrimethylene terephthalate (PTT)-based polyester.

Preferred in the present invention is a yarn of composite fibers in which the above two components are arranged in a side-by-side configuration or an eccentric core-sheath configuration along the longitudinal direction. Such a composite yarn is preferred because coil-shaped crimps can be formed by false twisting or heat treatment, thereby exhibiting excellent elasticity.

In the above combination of polymers, PTT and PET are preferably selected so that PTT has a high viscosity and PET has a low viscosity. In the process of spinning such polymers with different viscosities into a composite yarn with the configuration as described above, the stress concentrates on the high-viscosity polymer component, which leads to the difference in internal strain between the polymer components. Due to this difference, and in addition due to the difference in elastic recovery percentage in drawing and false-twisting process of the yarn and the difference in heat shrinkage rate in heat treatment of the resulting fabric, a large shrinkage occurs in the high-viscosity polymer component, thereby creating three-dimensional coil-shaped crimps. The diameter of the three-dimensional coils and the number of the coils per unit fiber length can be assumed to depend on the degree of difference in shrinkage between the high-shrinkage component and the low-shrinkage component (i.e., the sum total of the difference in the elastic recovery percentage and the difference in the heat shrinkage rate). A larger difference in shrinkage results in a smaller coil diameter and a larger number of the coils per unit fiber length.

The low-shrinkage component in the present invention is preferably PET. PET has advantages of having very good interfacial adhesion with the high-shrinkage component PTT and of being easily and stably melt-spun even in high-speed spinning at a speed of over 6000 m/min. In high-speed spinning of PTT, several problems may occur, for example, excessively tight winding may occur causing a difficulty in removing the package from the drum, or unevenness of a yarn in the longitudinal direction may occur resulting in poor quality. However, in the present invention, more than a certain ratio of PET is arranged as one of the two components in the composite spun yarn, and thereby excessively tight winding is prevented and deterioration of the quality of the wound package hardly occurs over time.

By using PET as one of the two components and adjusting the heat-setting temperature in the drawing and false-twisting process, the difference in the shrinkage rate between PET and the high-viscosity component PTT can be easily controlled. The heat shrinkage rate of PTT is hardly affected by the setting temperature, whereas the heat shrinkage rate of PET is largely affected by the setting temperature. Accordingly, when a high elasticity is desired, the difference in the heat shrinkage rate between PTT and PET is adjusted to be large, and the setting temperature in the drawing and false-twisting process is set at high temperature. On the other hand, when a low elasticity is desired, the difference in the shrinkage rate between PTT and PET is adjusted to be small, and the heat-setting temperature is set at low temperature.

The term "PTT" as used herein means a polyester produced by using terephthalic acid as a main acid component and 1,3-propanediol as a main glycol component.

The term "PET" as used herein means a polyester produced by using terephthalic acid as a main acid component and ethylene glycol as a main glycol component. The PTT and PET may contain 20 mol% of, preferably 10 mol% or less of, a copolymerization component that can form an ester bond. Examples of the copolymerizable compound include, but are not limited to, dicarboxylic acids, such as isophthalic acid, succinic acid, cyclohexanedicarboxylic acid, adipic acid, dimer acid, sebacic acid, and sodium 5-sulfoisophthalate; diols, such as ethylene glycol, propylene glycol, diethylene glycol, dipropylene glycol, butanediol, neopentyl glycol, cyclohexanedimethanol, polyethylene glycol, and polypropylene glycol.

Tubular woven construct contains two or more layers

The tubular woven construct of the present invention preferably contains two or more layers. The tubular woven construct containing two or more layers is advantageous in that the innermost layer of the tubular woven construct is protected from external force and that the durability is high. Such a tubular woven construct prevents leakage of a liquid or a powder during transportation or effectively protects linear bodies, such as wires, cables and conduits.

In particular cases where the tubular woven construct is used as a base material of a vascular prosthesis, the structure of the inner surface is adapted to be in contact with blood. When used as a base material of a vascular prosthesis, the tubular woven construct is preferably a multi-layer tubular woven fabric having a structure in which an outer-layer tubular woven fabric is superimposed on an inner-layer tubular woven fabric, and the inner-layer tubular woven fabric provides the inner surface to be in contact with blood, and the outer-layer tubular woven fabric provides the outer surface of the vascular prosthesis. The structure of the multi-layer tubular woven fabric used as a base material of a vascular prosthesis of the present invention may further contain a tubular woven fabric layer other than the inner-layer and outer-layer tubular woven fabrics. However, if the vascular prosthesis contains an excessively large number of tubular woven fabric layers, the vascular prosthesis may be excessively thick and the thickness may be largely different from that of the native blood vessel, which may reduce the efficiency of surgical operations, such as anastomosis, in implantation surgery. Accordingly, the number of tubular woven fabric layers is preferably from 2 to 4, more preferably 2 or 3.

The number of layers contained in the tubular woven construct of the present invention is not particularly limited. However, to achieve adequate elasticity and flexibility, the tubular woven construct is particularly preferably a double-weave woven construct formed by weaving two layers together by a known technique, such as connecting the inner layer to the outer layer by a binding warp yarn, connecting the inner layer to the outer layer by a binding weft yarn, and connecting the inner layer to the outer layer by binding weft yarns. Such a double-weave woven construct is advantageous in that there is no need for a bonding process of two woven fabrics by lamination or sewing, and moreover the two layers joined together by warp or weft threads can serve as a tubular woven construct with high flexibility and high mechanical strength.

In cases where the woven construct is a multi-layer woven construct containing two or more layers, the weft yarn is not particularly limited as long as it contains at least in part a microfiber yarn having a filament fineness of less than 1.0 dtex. The weft yarn can be appropriately selected from various types of synthetic fiber yarns made from synthetic resins depending on the purpose of use, and the weft yarn may be in any form of yarn selected, as appropriate, from multifilament yarns, monofilament yarns, etc. In particular cases where the woven construct is used as a base material of a vascular prosthesis, microfiber yarn threads having a filament fineness of less than 1.0 dtex are preferably disposed to form the inner surface of the vascular prosthesis.

The loom to be used may be a water-j et loom, an air-j et loom, a rapier loom, a shuttle loom, or the like. Of these, preferred is a shuttle loom, which is excellent in weaving a tubular fabric and can give a uniform tubular structure. The weave pattern of the double-weave vascular prosthesis may be plain weave, twill weave or sateen weave, or modified weave thereof, or multi-layer weave. The basic weaving process may be a known process.

Weft yarn in a layer other than the innermost layer contains at least in part a monofilament yarn having a thickness of 20 μm or more

The tubular woven construct of the present invention may be bent, or disposed in a meandering manner. The tubular woven construct of the present invention is excellent in kink resistance, and therefore flattening or twisting of the tubular woven construct hardly occurs, but preferably the kink resistance (bending resistance) is further enhanced. To this end, the weft yarn in a layer other than the innermost layer preferably contains at least in part a monofilament yarn having a thickness of 20 μm or more. Such a monofilament weft yarn is preferred because it is very stiff and imparts excellent kink resistance to the tubular woven construct.

The thickness of the monofilament yarn is more preferably 100 μm or more. The thickness is preferably up to 300 μm, more preferably up to 200 μm, for achieving adequate flexibility.

The monofilament yarn used in the present invention may be any type of organic fibers, but in order to achieve adequate water absorbability and degradation resistance, preferred are polyester fibers. Examples of the polyester include polyethylene terephthalate and polybutylene terephthalate. The monofilament yarn may be a monofilament yarn of copolymerized polyester produced by copolymerizing polyethylene terephthalate, polybutylene terephthalate, or the like with an acid component, for example, isophthalic acid, sodium 5-sulfoisophthalate, or an aliphatic dicarboxylic acid such as adipic acid. The monofilament yarn may be a monofilament yarn containing a core of polyethylene terephthalate and a sheath of copolymerized polyester having a lower melting point than the core. The monofilament yarn containing a low-melting point component in the sheath is preferred because the monofilament threads that form the outer surface of the tubular woven construct can be fused together by subsequent heat-setting treatment, thereby achieving stable mechanical strength, such as dimensional stability and kink resistance.

Water permeability of the inner surface of tubular woven fabric is 500 mL/min·120 mmHg (16 kPa)·$cm^2$ or less

An excessively large water permeability indicates that the size and number of the voids between the fibers are large. When a tubular woven construct with an excessively large water permeability is used as a hose for transporting a fluid or a powder, a large amount of leakage of a liquid or a powder may occur. When such a tubular woven construct is used as a vascular prosthesis, a large amount of leakage of blood may tend to occur. Therefore, the water permeability of the tubular woven construct of the present invention is preferably small.

In the present invention, the water permeability of the inner surface is preferably 500 mL/min·120 mmHg (16 kPa) ·$cm^2$ or less, more preferably 200 mL/min·120 mmHg·$cm^2$ or less, still more preferably 150 mL/min·120 mmHg·$cm^2$ or less.

In a conventional vascular prosthesis made of a common tubular woven fabric, a low water permeability cannot be achieved only by reducing the size and number of the voids between the fibers. Such a conventional vascular prosthesis requires attachment of a bioabsorbable gel, such as collagen and gelatin. In particular cases where such an attachment is performed on a conventional vascular prosthesis with a small luminal diameter, the fine structure formed by thin fibers with a small diameter and the voids between the fibers is destroyed and its ability to promote cell proliferation is diminished, resulting in a decrease in cellular affinity. In addition, the bioabsorbable gel, such as gelatin, rather attracts platelets and promotes adhesion of platelets to the surface of the gel, resulting in thrombus formation. The promotion of thrombus formation is especially noticeable in a vascular prosthesis with a luminal diameter of 6 mm or less. However, the tubular woven construct of the present invention has a low water permeability as described above in a preferred embodiment.

The water permeability of the inner surface of the tubular woven construct of the present invention for use as a vascular prosthesis, is preferably not less than 5 mL/min·120 mmHg (16 kPa) ·$cm^2$, more preferably not less than 10 mL/min ·120 mmHg·$cm^2$, still more preferably not less than 50 mL/min·120 mmHg·$cm^2$, to achieve biocompatibility of the vascular prosthesis.

The water permeability of the inner surface herein is determined as follows. The multi-layer tubular woven construct is closed at one end. From the other end, water at 25°C as sufficiently clean as tap water is fed into the woven construct for 20 minutes under the condition that the hydraulic pressure applied to the inner wall is 120 mmHg (16 kPa) . Then, the amount (mL) of water that leaks through the wall of the tubular woven construct per minute is measured. The measured amount is divided by the surface area ($cm^2$) of the multi-layer tubular woven construct. The thus determined water permeability can be used as an index showing the size and number of the voids between the fibers of the vascular prosthesis. The water permeability can be adjusted by adjusting the proportion of the warp and weft threads that form the tubular woven construct whose inner surface is to be in contact with blood, the diameter of a filament of the warp and weft yarns, the packing density of the warp and weft threads, the thickness and hydrophilicity of an antithrombogenic material layer, etc.

Antithrombogenicity

A tubular woven fabric implanted as a vascular prosthesis is typically recognized as foreign by the body. In particular, blood coagulation reaction proceeds on the surface in contact with blood, i.e., the inner surface of the vascular prosthesis, leading to the formation of thrombi. In order to prevent this, antithrombogenicity is preferably imparted to the vascular prosthesis. Antithrombogenicity of a medical material can conventionally be enhanced by attaching heparin or a heparin derivative to a surface of the material. However, heparin or a heparin derivative cannot be directly attached to particular types of vascular prostheses, such as a vascular prosthesis made of a medical textile material made of polyester fibers etc. and a vascular prosthesis made of a medical material made of porous expanded polytetrafluoroethylene (hereinafter referred to as "ePTFE"). To overcome this problem, there have been proposed methods to covalently bind heparin or a heparin derivative to a modified surface of a medical material (Patent Literature 11 to 13), and methods to ionically bind heparin or a heparin derivative to a surface of a material (Patent Literature 14 to 17) .

Several other methods for imparting antithrombogenicity to a textile vascular prosthesis have also been proposed, including a method in which heparin or a heparin derivative is added to a bioabsorbable gel used for prevention of leakage of blood, such as collagen and gelatin, and the heparin-containing gel is attached to a surface of a material (Patent Literature 18) ; and a method in which a segmented polyurethane dissolved in an organic solvent is impregnated into a material, thereby attaching the polyurethane on a surface of the material (Patent Literature 19).

Methods for enhancing antithrombogenicity of a medical material by using an antithrombogenic compound other than heparin or a heparin derivative have also been proposed, including a method in which a particular compound is attached to a surface of a medical material. The compound is exemplified by a compound that inhibits blood coagulation factors involved in the blood coagulation reaction (e.g., platelets, which are involved in the primary hemostasis) , a compound that inhibits thrombin, which is involved in the thrombus formation (Patent Literature 20 to 22).

Such antithrombogenic treatment may be applied to the tubular woven construct of the present invention. The

tubular woven construct that has been subjected to the antithrombogenic treatment is capable of preventing adherence of thrombi and of being implanted in a living body for a long period of time, and is therefore suitable a vascular prosthesis. The antithrombogenic material that can be provided to the tubular woven construct is preferably as described below.

Antithrombogenic material

The antithrombogenicity is a property that prevents blood coagulation on a surface in contact with blood. In particular, the antithrombogenicity refers to, for example, a property that inhibits platelet aggregation or blood coagulation, which proceeds by activation of blood coagulation factors such as thrombin. The cellular affinity is, in particular, the affinity for vascular endothelial cells, which are present on the inner surface of blood vessels of a living body and serve to inhibit thrombus formation, and the cellular affinity refers to a property that promotes the settlement of vascular endothelial cells, thereby promoting intimal formation.

The antithrombogenic material is a material having antithrombogenicity. In particular, preferred antithrombogenic materials in the present invention are an antithrombogenic material A containing a sulfur-containing anionic compound having anticoagulant activity and a cationic polymer; and an antithrombogenic material B containing the following three types of skeletal structures: a hydrophilic polymer skeleton, a 4-(aminomethyl)benzenecarboxyimidamide or benzamidine skeleton, and a methoxy benzene sulfonamide skeleton.

In the vascular prosthesis of the present invention, the antithrombogenic material is preferably bound to the tubular woven construct's inner surface to be in contact with blood, thereby forming an antithrombogenic material layer. When the antithrombogenic material layer on the inner surface of the vascular prosthesis to be in contact with blood has an excessively large thickness, the antithrombogenic material layer may destroy the fine structure of the inner surface formed of the warp and weft yarns that form the tubular woven construct of the present invention having the blood-contacting inner surface, and as a result, the settlement of vascular endothelial cells tends to be less likely to occur. On the other hand, when the antithrombogenic material layer has an excessively small thickness, the amount of binding of the antithrombogenic material may be small, and as a result, the optimum antithrombogenicity is less likely to be exhibited immediately after implantation of the vascular prosthesis. In other words, the antithrombogenic material layer is preferably formed so as to have an appropriate thickness by binding of the antithrombogenic material to the inner surface of the tubular woven construct to be in contact with blood. Specifically, the thickness is preferably from 1 to 600 nm, more preferably from 5 to 500 nm, still more preferably from 15 to 400 nm.

The thickness of the antithrombogenic material layer can be determined by, for example, using a scanning transmission electron microscope as described later (hereinafter referred to as "STEM"). The thickness of the antithrombogenic material layer is, when the atomic distribution in the layer is measured in the vertical direction from the inner surface toward the outer surface using a STEM, a distance between the start and end points where the atoms derived from the antithrombogenic material are observed. The thickness is measured at at least three randomly selected positions and the measured values are averaged to determine the mean thickness.

When the tubular woven construct of the present invention is used as a vascular prosthesis, the antithrombogenic material is preferably distributed toward the outer layer of the tubular woven construct having the blood-contacting inner surface, i.e., in the depth direction in the STEM measurement. The inner surface subjected to the STEM measurement refers to a portion of the inner surface subjected to the analysis of the atomic distribution in the vertical direction from the inner surface toward the outer surface, in particular, a region extending from the fabric's blood-contacting inner surface in contact with an embedding resin used for STEM sample preparation (e.g., an acrylic resin) toward the outer surface of the fabric. Specifically, the starting point of the thickness measurement of the antithrombogenic material layer is not exactly on the inner surface subjected to the STEM analysis, but is a point in the fabric's warp and weft yarns where the atoms derived from the antithrombogenic material are observed. The distance between the start and end points where the atoms derived from the antithrombogenic material layer are observed is preferably 15 nm or more, that is, the antithrombogenic material layer preferably extends for 15 nm or more in the depth direction from the inner surface of the construct. When the distance between the start and end points where the atoms derived from the antithrombogenic material are observed is less than 15 nm, the amount of binding of the antithrombogenic material is small and insufficient to exhibit the desired antithrombogenicity required immediately after implantation. The distance between the start and end points where the atoms derived from the antithrombogenic material are observed may be more than 200 nm, but the distance should be less than this length. The reason of this is that, in order to allow the presence of the atoms derived from the antithrombogenic material in the outer layer, i.e., toward the outer layer exceeding the above length (i.e., to allow the distribution of the antithrombogenic material in the depth direction exceeding the above length), the constituent fibers of the vascular prosthesis are required to be subjected to hydrolysis and oxidation treatment with an acid or alkali and an oxidant to a degree appropriate for the above length, which may deteriorate the vascular prosthesis resulting in a decrease in the mechanical characteristics, such as tensile strength. Accordingly, in the present invention, the antithrombogenic material is preferably bound to the warp and weft yarns that form the tubular woven construct having the blood-contacting inner surface in such a manner that the end point where the atoms derived from the antithrombogenic

material are observed will be 15 to 200 nm in depth.

As described above, we found as a result of extensive research that the settlement of vascular endothelial cells and the formation of the intima can be more effectively promoted on the blood-contacting inner surface of the vascular prosthesis by providing an antithrombogenic material layer of an appropriate thickness formed through binding the antithrombogenic material to the warp and the weft yarns that form the tubular woven construct having the blood-contacting inner surface, without destroying the fine structure of the inner surface formed of the warp and weft yarns. We also found that, since a sufficient amount of the antithrombogenic material can be bound to the warp and the weft yarns without destroying the fine structure, the desired antithrombogenicity can be exhibited immediately after implantation, and thus both high antithrombogenicity and high cellular affinity can be achieved.

Specifically, the thickness of the antithrombogenic material layer, or the distance between the start and end points where the atoms derived from the antithrombogenic material are observed when the atomic distribution in the warp and weft yarns that form the tubular woven construct having the blood-contacting inner surface is measured in the vertical direction from the inner surface toward the outer surface, can be determined by a combination of, for example, STEM analysis and X-ray photoelectron spectroscopy (hereinafter referred to as "XPS"). A STEM detector is, for example, an energy dispersive X-ray spectrometer (hereinafter referred to as "EDX") or an electron energy-loss spectrometer (hereinafter referred to as "EELS"). The measurement conditions for STEM are as follows.

Measurement conditions

Apparatus: field emission transmission electron microscope JEM-2100F (JEOL Ltd.)
EELS detector: GIF Tridiem (GATAN, Inc.)
EDX detector: JED-2300T (JEOL Ltd.)
Image acquisition: Digital Micrograph (GATAN, Inc.)
Sample preparation: ultramicrotomy (the samples are embedded in an acrylic resin, and the sliced sections are placed on a copper microgrid.)
Accelerating voltage: 200 kV
Beam diameter: 0.7 nm
Energy resolution: about 1.0 eVFWHM

The existence of a particular atom is confirmed from the presence of a peak corresponding to the atom in a spectrum obtained by STEM measurement after subtraction of the background.

The antithrombogenic material A is preferably a sulfur-containing anionic compound having anticoagulant activity. The antithrombogenic material A preferably further contains a cationic polymer, in particular and more preferably, a cationic polymer containing, as a constituent monomer A, a compound selected from the group consisting of alkyleneimines, vinyl amines, allylamine, lysine, protamines, and diallyl dimethyl ammonium chloride.

These constituent monomers A have a cationic nitrogen atom, and their polymers are cationic. On the other hand, the sulfur-containing compound having anticoagulant activity is anionic, and can therefore bind to the cationic polymer by ionic bonding. Examples of the sulfur-containing anionic compound having anticoagulant activity include heparin and heparin derivatives, dextran sulfate, polyvinyl sulfonate, and polystyrene sulfonate. Preferred are heparin and heparin derivatives. The heparin and heparin derivatives may be purified or unpurified, and are not particularly limited as long as they inhibit blood coagulation reaction. Examples of the heparin and heparin derivatives include heparin that is commonly clinically applied, unfractionated heparin, low-molecular-weight heparin, and heparin with high affinity to antithrombin III. Specific examples of heparin include "heparin sodium" (Organon API, Inc.) etc.

The cationic polymer has cationic properties and may exhibit hemolytic toxicity etc. Therefore, elution of the polymer into the blood is not preferred. Thus, the cationic polymer is preferably bound to, more preferably covalently bound to, the warp and weft yarns that form the tubular woven construct having the blood-contacting inner surface.

The cationic polymer may be a homopolymer or a copolymer. When the cationic polymer is a copolymer, the copolymer may be any of a random copolymer, a block copolymer, a graft copolymer, and an alternating copolymer. Of these, a block copolymer containing successively repeating units containing a nitrogen atom is more preferred because strong ionic bonding can be formed by interaction between the blocks and the sulfur-containing anionic compound having anticoagulant activity.

The term "homopolymer" as used herein means a macromolecular compound obtained by polymerization of a single type of constituent monomer. The term "copolymer" as used herein means a macromolecular compound obtained by copolymerization of two or more types of monomers. The term "block copolymer" as used herein means a copolymer having a molecular structure in which at least two types of polymers having different repeating units are covalently bound to each other to form a longer chain. The term "block" as used herein means each of at least two types of polymers

constituting the block copolymer, the constituting polymers having different repeating units.

The cationic polymer herein may be linear or branched, but the branched polymer is preferred because the branched polymer can form a large number of more stable ionic bonds with the sulfur-containing anionic compound having anticoagulant activity.

The cationic polymer herein has at least one functional group selected from primary to tertiary amino groups and a quaternary ammonium group. In particular, the cationic polymer having a quaternary ammonium group is more preferred because a quaternary ammonium group forms stronger ionic interaction with the sulfur-containing anionic compound having anticoagulant activity than primary to tertiary amine groups, and hence allows easier control of the elution rate of the sulfur-containing anionic compound having anticoagulant activity.

The number of carbon atoms in the three alkyl groups of the quaternary ammonium group are not particularly limited in the present invention. However, when the number of carbon atoms contained in the three alkyl groups is excessively large, the quaternary ammonium group is highly hydrophobic, and steric hindrance is large. Consequently, the quaternary ammonium group cannot effectively bind, by ionic bonding, to the sulfur-containing anionic compound having anticoagulant activity. Another disadvantage is that, when the number of carbon atoms is excessively large, the polymer is more likely to exhibit hemolytic toxicity. The number of carbon atoms contained in a single alkyl group bound to the nitrogen atom of the quaternary ammonium group is preferably from 1 to 12, more preferably from 2 to 6. The number of carbon atoms contained in each of the three alkyl groups bound to the nitrogen atom of the quaternary ammonium group may be the same as or different from each other.

The cationic polymer is preferably a polyalkyleneimine in the present invention. Use of a polyalkyleneimine as the cationic polymer is advantageous because the amount of the sulfur-containing anionic compound having anticoagulant activity adsorbed to the cationic polymer by ionic interaction becomes large. Examples of the polyalkyleneimine include polyethyleneimine (hereinafter referred to as "PEI"), polypropyleneimine, polybutyleneimine, and alkoxylated polyalkyleneimine. More preferred is PEI.

Specific examples of the PEI include "LUPASOL" (registered trademark) (BASF SE), and "EPOMIN" (registered trademark) (Nippon Shokubai Co., Ltd.). The PEI may be a copolymer with one or more other monomers or a modified PEI polymer as long as the effects of the present invention are not deteriorated. The term "modified polymer" as used herein means a polymer that has the same constituent monomers A as in the original cationic polymer but has partially undergone, for example, radical decomposition or recombination by irradiation as described later.

A constituent monomer of the cationic copolymer other than alkyleneimines, vinyl amines, allylamines, lysine, protamines, or diallyl dimethyl ammonium chloride is not particularly limited, and may be, for example, ethylene glycol, propylene glycol, vinylpyrrolidone, vinyl alcohol, vinyl caprolactam, vinyl acetate, styrene, methyl methacrylate, hydroxyethyl methacrylate, or siloxane, which is designated herein as a constituent monomer B. An excessively large amount of the constituent monomer B by weight may result in the tendency of weak ionic bonding between the cationic polymer and the sulfur-containing anionic compound having anticoagulant activity. Thus the amount by weight of the constituent monomer B is preferably 10% by weight or less.

If the weight average molecular weight of the cationic polymer is excessively small in the present invention, the molecular weight tends to be smaller than that of the sulfur-containing anionic compound having anticoagulant activity, and consequently stable ionic bonds cannot be formed and as a result, the desired antithrombogenicity is less likely to be achieved. On the other hand, if the weight average molecular weight of the cationic polymer is excessively large, the sulfur-containing anionic compound having anticoagulant activity is encapsulated in the cationic polymer, and consequently the antithrombogenic moiety tends to be embedded in the cationic polymer. Thus, the weight average molecular weight of the cationic polymer is preferably 600 to 2, 000, 000, more preferably 1,000 to 1,500,000, still more preferably 10,000 to 1,000,000. The weight average molecular weight of the cationic polymer can be measured by, for example, gel permeation chromatography or the light scattering method.

We conducted extensive research in connection with the present invention to achieve both high antithrombogenicity and high cellular affinity due to the presence of the sulfur-containing anionic compound having anticoagulant activity, without destroying the fine structure formed of the warp yarn and the weft yarn containing a microfiber yarn having a filament fineness of less than 1.0 dtex in the tubular woven construct having the blood-contacting inner surface. As a result, we found that there is a preferred value for the abundance ratio of sulfur atoms relative to all the atoms on the inner surface as measured by XPS. The abundance ratio of a particular atom is expressed in terms of the "atomic percent", which gives the percentage of abundance of a particular atom relative to all the atoms, taken as 100.

The abundance ratio of sulfur atoms relative to all the atoms on the inner surface as measured by XPS in the present invention is preferably from 3.0 to 6.0 atomic percent, more preferably from 3.2 to 5.5 atomic percent, still more preferably from 3.5 to 5.0 atomic percent. When the abundance ratio of sulfur atoms relative to all the atoms is less than 3.0 atomic percent, the amount of binding of the sulfur-containing anionic compound having anticoagulant activity is small, and therefore good antithrombogenicity is less likely to be exhibited immediately after implantation of the vascular prosthesis. On the other hand, when the abundance ratio of sulfur atoms relative to all the atoms is more than 6.0 atomic percent, the amount of binding of the sulfur-containing anionic compound having anticoagulant activity is sufficient, and

therefore the desired antithrombogenicity can be obtained, but a large amount of the cationic polymer is required to be ionically bound to the anionic compound and to be covalently bound to the warp and weft yarns that form the tubular woven construct having the blood-contacting inner surface. In addition, as elution of the anionic compound proceeds, the cationic polymer becomes exposed and may exhibit hemolytic toxicity etc. For these reasons, it is not preferred that the abundance ratio of sulfur atoms relative to all the atoms exceeds 6.0 atomic percent.

When the abundance ratio of sulfur atoms relative to all the atoms is 6.0 atomic percent or less, the amount of binding of the sulfur-containing anionic compound having anticoagulant activity is appropriate, leading to promotion of the settlement of vascular endothelial cells.

Specifically, the abundance ratio of sulfur atoms relative to all the atoms on the inner surface can be determined by XPS.

<u>Measurement conditions</u>

Apparatus: ESCALAB 220iXL (VG Scientific)
Excitation X-ray: monochromatic AlK$\alpha$1, $\alpha$2 radiation (1486.6 eV)
X-ray beam diameter: 1 mm
X-electron take-off angle: 90° (the angle of the detector relative to the surface of the vascular prosthesis)

The inner surface to be subjected to measurement by X-ray photoelectron spectroscopy (XPS) is the inner surface of the vascular prosthesis that has been cut open. In particular, the inner surface to be subjected to the measurement refers to a region extending from the measurement surface to a depth of 10 nm in the XPS measurement under the conditions that the X-electron take-off angle, i.e., the angle of the detector with respect to the inner surface of the vascular prosthesis in which the antithrombogenic material is bound to the tubular woven fabric is 90°. The fibers in the tubular woven construct of the present invention may contain sulfur atoms that are not derived from the antithrombogenic material, or may contain no sulfur atoms.

By radiating X-rays to the inner surface of the vascular prosthesis and measuring the energy of photoelectrons generated therefrom, the binding energy of the bound electrons in the material can be determined. From the binding energy, the information on the atoms on the inner surface subjected to XPS measurement can be obtained, and from the shift in binding energy peaks, the information on the valence and the binding state can be obtained. From the area ratio of each peak, quantification can be performed, i.e., the abundance ratios of atoms, valence, and binding state can be calculated.

Specifically, the S2p peak, which indicates the presence of sulfur atoms, is observed at a binding energy of around 161 eV to around 170 eV. We found that the ratio of area of the S2p peak to the total peak area is preferably from 3.0 to 6.0 atomic percent. In the calculation of the abundance ratio of sulfur atoms relative to all the atoms, the value is rounded to one decimal place.

We also found that there is a preferred value for the abundance ratio of nitrogen atoms relative to all the atoms on the inner surface as measured by XPS. The abundance ratio of nitrogen atoms relative to all the atoms on the inner surface as measured by XPS is preferably from 6.0 to 12.0 atomic percent, more preferably from 7.0 to 12.0 atomic percent, still more preferably from 7.5 to 11.0 atomic percent, still more preferably from 8.0 to 10.0 atomic percent. When the abundance ratio of nitrogen atoms relative to all the atoms is less than 6.0 atomic percent, the amount of the cationic polymer bound to the tubular woven construct having the blood-contacting inner surface is small. In such cases, the tubular woven construct having the blood-contacting inner surface maintains the fine structure formed of the warp yarn and the weft yarn containing a microfiber yarn having a filament fineness of less than 1 dtex, but the amount of the sulfur-containing anionic compound having anticoagulant activity bound to the cationic polymer by ionic bonding is small, and as a result, the optimum antithrombogenicity is less likely to be exhibited immediately after implantation of the vascular prosthesis. On the other hand, when the abundance ratio of nitrogen atoms relative to all the atoms is more than 12.0 atomic percent, the amount of the cationic polymer bound to the tubular woven construct having the blood-contacting inner surface is large. In such cases, the amount of the sulfur-containing anionic compound having anticoagulant activity bound to the cationic polymer by ionic bonding is sufficient, but it was found that, as elution of the anionic compound proceeds, a large amount of the cationic polymer becomes exposed and exhibits hemolytic toxicity.

When the abundance ratio of nitrogen atoms relative to all the atoms is 12.0 atomic percent or less, the amount of binding of the sulfur-containing anionic compound having anticoagulant activity is appropriate, leading to promotion of the settlement of vascular endothelial cells. In order to achieve both antithrombogenicity and cellular affinity, the abundance ratio of nitrogen atoms relative to all the atoms is preferably from 6.0 to 12.0 atomic percent, more preferably from 6.0 to 9.5 atomic percent, still more preferably from 8.0 to 9.5 atomic percent.

Specifically, the N1s peak, which indicates the presence of nitrogen atoms, is observed at a binding energy of

around 396 eV to around 403 eV. We found that the ratio of area of the N1s peak to the total peak area is preferably from 7.0 to 12.0 atomic percent in the present invention. The N1s peak can be split into two components, i.e., the main N1 component (at around 399 eV), which is attributed to carbon-nitrogen (hereinafter referred to as "C-N") bonds; and the N2 component (at around 401 to 402 eV), which is attributed to an ammonium salt, C-N (in a different structure from that of N1), and/or nitrogen oxide (hereinafter referred to as "NO"). The abundance ratio of each of the split peak components can be calculated according to Equation 2 below. In the calculation, the abundance ratio of nitrogen atoms relative to all the atoms and the abundance ratio of each split peak component are rounded to one decimal place.

$$Split_{ratio} = N1s_{ratio} \times (split_{percent}/100) \quad \text{(Equation 2)}$$

$Split_{ratio}$: the abundance ratio (%) of each split peak component
$N1s_{ratio}$: the abundance ratio (%) of nitrogen atoms to all the atoms
$Split_{percent}$: the abundance ratio (%) of each split peak component in the N1s peak

The N2 component, which is attributed to NO, obtained by splitting the N1s peak indicates the presence of quaternary ammonium groups in the present invention. We found that the abundance ratio of the N2 component to the all the components of the N1s peak, i.e., $Split_{percent}$ (N2), is preferably from 20 to 70 atomic percent, more preferably from 25 to 65 atomic percent, still more preferably from 30 to 60 atomic percent. When $Split_{percent}$ (N2) is less than 20 atomic percent, the abundance of quaternary ammonium groups is low. Consequently, the ionic interaction with the sulfur-containing anionic compound having anticoagulant activity is weak, which accelerates the elution of the anionic compound, and as a result, the optimum antithrombogenicity is less likely to be exhibited immediately after implantation of the vascular prosthesis. On the other hand, when $Split_{percent}$ (N2) is more than 70 atomic percent, the ionic interaction with the sulfur-containing anionic compound having anticoagulant activity tends to be excessively strong. In such cases, because of a decrease in the degree of freedom due to formation of an ionic complex, high anticoagulant activity cannot be maintained for a long period of time, and the elution rate tends to be low. Because of the above reasons, the abundance ratio of the N2 component, i.e., $Split_{ratio}$ (N2), which is calculated according to Equation 2, is preferably from 1.4 to 8.4 atomic percent, more preferably from 1.8 to 7.2 atomic percent, still more preferably from 2.4 to 6.0 atomic percent.

The C1s peak, which indicates the presence of carbon atoms, is observed at a binding energy of around 282 to 292 eV. The C1s peak can be split into five components, i.e., the main C1 component (at around 285 eV), which is attributed to carbon-hydrogen (hereinafter referred to as "CHx") bonds suggesting the presence of a saturated hydrocarbon(s) etc., to carbon-carbon (hereinafter referred to as "C-C") bonds, and/or to carbon=carbon (hereinafter referred to as "C=C") bonds; the C2 component (at around 286 eV), which is attributed to carbon-oxygen (hereinafter referred to as "C-O") bonds suggesting the presence of an ether (s) and/or hydroxyl groups, and/or to carbon-nitrogen (hereinafter referred to as "C-N") bonds; the C3 component (at around 287 to 288 eV), which is attributed to carbon=oxygen (hereinafter referred to as "C=O") bonds suggesting the presence of carbonyl groups; the C4 component (at around 288 to 289 eV), which is attributed to oxygen=carbon-oxygen (hereinafter referred to as "O=C-O") bonds suggesting the presence of ester groups and/or carboxyl groups; and the C5 component (at around 290 to 292 eV), which is attributed to $\pi$-$\pi$* satellite peak (hereinafter referred to as "$\pi$-$\pi$") bonds suggesting the presence of a conjugated system(s) such as benzene rings. The abundance ratio of each of the split peak components can be calculated according to Equation 3 below. In the calculation, the abundance ratio of carbon atoms relative to all the atoms and the abundance ratio of each split peak component are rounded to one decimal place.

$$Split_{ratio} = C1s_{ratio} \times (split_{percent}/100) \quad \text{(Equation 3)}$$

$Split_{ratio}$: the abundance ratio (%) of each split peak component
$C1s_{ratio}$: the abundance ratio (%) of carbon atoms to all the atoms
$Split_{percent}$: the abundance ratio (%) of each split peak component in the C1s peak

The C3 component, which is attributed to C=O bonds, obtained by splitting the C1s peak indicates the presence of amide groups in the present invention. We found that the abundance ratio of the C3 component to all the components of the C1s peak, i.e., the abundance ratio of amide groups, is preferably 2.0 atomic percent or more, more preferably 3.0 atomic percent or more. When the abundance ratio of the amide groups is less than 2.0 atomic percent, the number of covalent amide bonds formed between the cationic polymer and the tubular woven construct having the blood-contacting inner surface is small. Consequently, the amount of binding of the cationic polymer is small, and the ionic bonding between the cationic polymer and the sulfur-containing anionic compound having anticoagulant activity is weak. Thus, the optimum antithrombogenicity is less likely to be obtained.

The antithrombogenic material B preferably contains the following three types of skeletal structures: a hydrophilic polymer skeleton, a 4- (aminomethyl) benzenecarboxyimidamide or benzamidine skeleton, and a methoxy benzene sulfonamide skeleton. Specifically, the hydrophilic polymer skeleton preferably contains, as a constituent monomer B, a compound selected from the group consisting of ethylene glycol, propylene glycol, vinylpyrrolidone, vinyl alcohol, vinyl caprolactam, vinyl acetate, styrene, methyl methacrylate, hydroxyethyl methacrylate, and siloxane.

The three types of skeletal structures may be separately contained in different compounds, or at least two of the skeletal structures may be combined by covalent or ionic bonds into a single compound. The antithrombogenic material B is preferably a compound containing all the following three types of skeletal structures: the hydrophilic polymer skeleton, the 4-(aminomethyl)benzenecarboxyimidamide or benzamidine skeleton, and the methoxy benzene sulfonamide skeleton. Such a compound is advantageous in achieving both antithrombogenicity and cellular affinity in the vascular prosthesis of the present invention.

At least one of the three types of skeletal structures preferably contains a functional group selected from the group consisting of, for example, hydroxyl, thiol, amino, carboxyl, aldehyde, isocyanate, and thioisocyanate groups, more preferably contains an amino or carboxyl group, still more preferably contains an amino group. The functional group is preferably contained in the hydrophilic polymer skeleton, and is more preferably present at an end of the hydrophilic polymer skeleton. By using one or more functional groups selected from the group consisting of hydroxyl, thiol, amino, carboxyl, aldehyde, isocyanate, and thioisocyanate groups, the warp and weft yarns that form the tubular woven construct having the blood-contacting inner surface can be covalently bound to the three types of skeletal structures via, for example, disulfide bonds, amide bonds, ester bonds, urethane bonds, bonds by condensation reaction, and/or the like.

The one or more reactive functional groups contained in the antithrombogenic material allow covalent bonding of the antithrombogenic material to the warp and weft yarns that form the tubular woven construct having the blood-contacting inner surface. Therefore, irradiation or other methods are not required to form covalent bonds . When covalent bonds are formed by irradiation or other methods as described in Patent Literature 15 and 16, the 4-(aminomethyl)benzenecarboxyimidamide or benzamidine skeleton and the methoxy benzene sulfonamide skeleton absorb high energy from the radiation and generate highly reactive radicals. The generated radicals react with a site in the compound and change the skeletal structures, mainly leading to a decrease in anti-thrombin activity.

We also found that the hydrophilic polymer skeleton is important to enhance anti-platelet adhesion activity associated with the antithrombogenicity during our extensive research to achieve both high antithrombogenicity and high cellular affinity in the antithrombogenic material B, which is preferred in the present invention.

The hydrophilic polymer skeleton is a polymer skeleton containing hydrophilic functional groups and having solubility in water. The hydrophilic polymer may be a copolymer with one or more other monomers or a modified polymer as long as the effects of the present invention are not deteriorated.

The hydrophilic polymer skeleton may be a homopolymer or a copolymer as long as it contains one or more constituent monomers B as described above. When the hydrophilic polymer is a copolymer, the copolymer may be any of a random copolymer, a block copolymer, a graft copolymer, and an alternating copolymer. The hydrophilic polymer skeleton may be linear or branched.

We also found that the 4-(aminomethyl)benzenecarboxyimidamide or benzamidine skeleton and the methoxy benzene sulfonamide skeleton are important to enhance anti-thrombin activity associated with the antithrombogenicity during our extensive research to achieve both antithrombogenicity and cellular affinity in the antithrombogenic material B, which is preferred in the present invention.

The 4-(aminomethyl)benzenecarboxyimidamide skeleton is any of the skeletal structures represented by general formula (V). The benzamidine skeleton is any of the skeletal structures represented by general formula (VI). The methoxy benzene sulfonamide skeleton is any of the skeletal structures represented by general formula (VII).

(V)

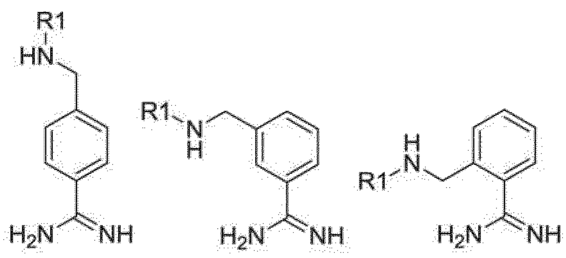

(In the formulae, R1 is a moiety linked to another skeletal structure.)

(VI)

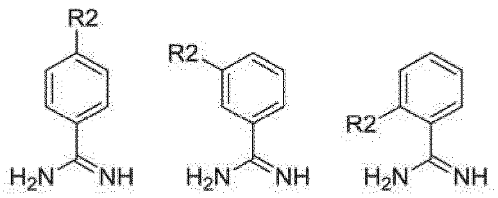

(In the formulae, R2 is a moiety linked to another skeletal structure.)

(VII)

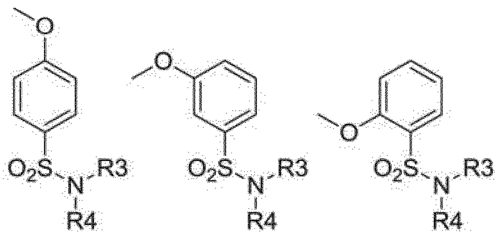

(In the formulae, R3 and R4 each are a moiety linked to another skeletal structure.)

A preferred compound containing all the following three types of skeletal structures: the hydrophilic polymer skeleton, the 4-(aminomethyl)benzenecarboxyimidamide or benzamidine skeleton, and the methoxy benzene sulfonamide skeleton is any of the compounds represented by general formulae (I) to (IV). In these general formulae, X is preferably an amino or carboxyl group, and is more preferably an amino group.

(I)

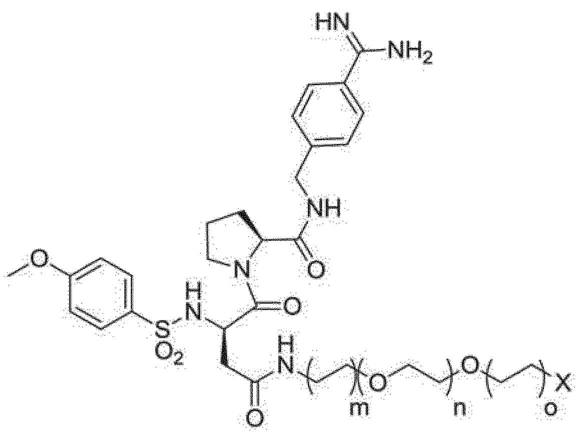

(II)

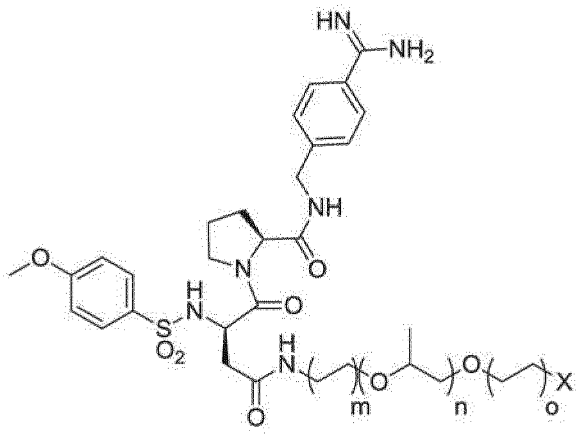

(III)

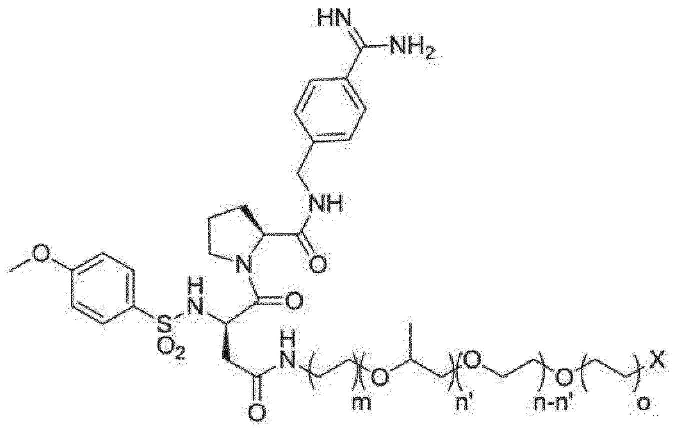

(IV)

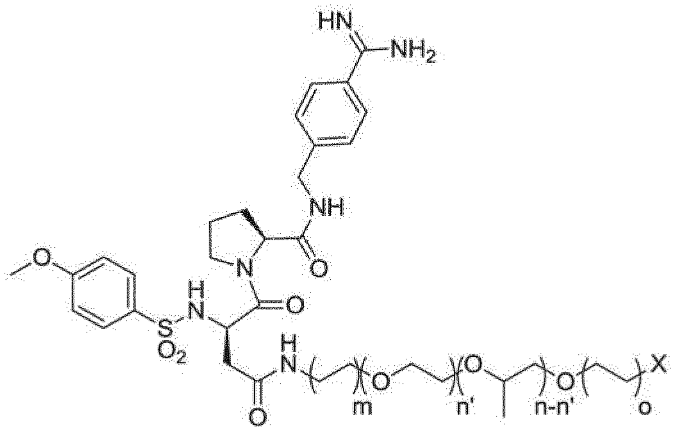

(In the formulae (I) to (IV), m and o each represent an integer of 0 to 4; n represents an integer of 3 to 1000, and n' represents an integer of 3 to 1000, with the proviso that n and n' satisfy the formula: $n \geq n'$ ; and X represents a functional group selected from the group consisting of hydroxyl, thiol, amino, carboxyl, aldehyde, isocyanate, and thioisocyanate groups.)

X in the formulae may be contained in any of the three types of skeletal structures in the present invention. In our findings, when the hydrophilic polymer skeleton, which has anti-platelet adhesion activity, is present on the fabric-neighboring side of the antithrombogenic material layer, and the 4-(aminomethyl)benzenecarboxyimidamide or benzamidine skeleton and the methoxy benzene sulfonamide skeleton, which have anti-thrombin activity, are present on the opposite side to be in contact with blood, the latter skeletons exhibit higher thrombin capture activity, and consequently higher and longer-lasting antithrombogenicity can be exhibited. Based on the findings, the reactive functional group (X in the above formulae) to be covalently bound to the warp and weft yarns that form the tubular woven construct is preferably contained in the hydrophilic polymer skeleton, and is more preferably present at the end of the hydrophilic polymer skeleton. By using the reactive functional group X in the formulae, the warp and weft yarns that form the tubular woven construct having the blood-contacting inner surface can be covalently bound to the compounds in the antithrombogenic material via, for example, disulfide bonds, amide bonds, ester bonds, urethane bonds, bonds by condensation reaction, and/or the like.

The term "bond" as used herein means a chemical bond, such as a covalent bond, a hydrogen bond, an ionic bond, or a coordinate bond. The term "covalent bond" means a chemical bond formed by sharing of electrons between atoms. Examples of the types of covalent bonds include, but are not limited to, an amine bond, an azide bond, an amide bond, and an imine bond. Of these, an amide bond is preferred because the covalent bond is easily formed and the bond has high stability. The formation of covalent bonds can be confirmed through observation of no elution after washing of the vascular prosthesis with a solvent that dissolves the antithrombogenic material.

We found in connection with the present invention that, for maintenance of higher and longer-lasting antithrombogenicity, the antithrombogenic material B more preferably contains a betaine compound, and the betaine compound is covalently bound to the warp and weft yarns that form the tubular woven construct having the blood-contacting inner surface, or to the antithrombogenic material B.

The term "betaine compound" means a compound that has positive and negative charges that are not adjacent to each other in a single molecule, in which a positively charged atom has no dissociable hydrogen and which molecule is neutral as a whole; or a salt thereof. The betaine compound herein is not particularly limited as long as it contains a betaine moiety in the molecule, but is preferably carboxybetaine, sulfobetaine, or phosphobetaine, and is more preferably carboxybetaine or sulfobetaine represented by general formula (VIII) or (IX). In general formulae (VIII) and (IX), X is preferably an amino or carboxyl group, and is more preferably an amino group.

(VIII)

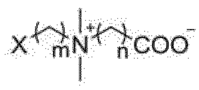

(IV)

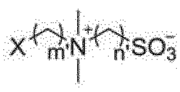

(In the formulae (VIII) and (IX), n represents an integer of 1 to 4; m represents an integer of 2 to 4; n' represents an integer of 2 to 4; m' represents an integer of 2 to 4; and X represents a functional group selected from the group consisting of hydroxyl, thiol, amino, carboxyl, aldehyde, isocyanate, and thioisocyanate groups.)

The presence of the hydrophilic polymer skeleton, the 4-(aminomethyl)benzenecarboxyimidamide or benzamidine skeleton and the methoxy benzene sulfonamide skeleton on the innermost surface of the vascular prosthesis to which the antithrombogenic material B is bound, which material is preferred in the present invention, can be determined by time-of-flight secondary ion mass spectrometry (hereinafter referred to as "TOF-SIMS").

<u>Measurement conditions</u>

Apparatus: TOF.SIMS 5 (ION-TOF GmbH)
Primary ion species: $Bi^{3++}$
Secondary ion polarity: positive and negative
Mass range (m/z): 0 to 1500
Raster size: 300 μm × 300 μm
Number of pixels (on each side): 256 pixels

Post acceleration: 10 kV
Degree of vacuum for measurement (before sample injection) : $4 \times 10^{-7}$ Mpa
Acceleration voltage of primary ions: 25 kV
Pulse width: 10.5 ns
Bunching: yes (high mass resolution)
Charge neutralization: yes

The "TOF-SIMS" measurement herein is performed on the inner layer of the vascular prosthesis that has been cut open. The "innermost surface subjected to the TOF-SIMS measurement" is a region extending from the measurement surface to a depth of 1 to 3 nm in the TOF-SIMS measurement under the above conditions .

The innermost surface to be subjected to TOF-SIMS measurement is placed under ultrahigh vacuum and bombarded with pulsed primary ions, then secondary ions having a certain amount of kinetic energy are extracted from the innermost surface, and the secondary ions are guided to the time-of-flight mass spectrometer. The obtained mass spectrum reflects the mass of the secondary ions. Based on the mass spectrum, organic and inorganic substances present on the innermost surface subjected to TOF-SIMS measurement can be identified, and information on the abundance of each substance can be obtained from the peak intensities.

Specifically, the presence of an ethylene glycol skeleton or a propylene glycol skeleton on the innermost surface subjected to TOF-SIMS measurement can be confirmed from at least one peak selected from the group consisting of $^{45}C_2H_5O^+$, $^{59}C_3H_7O^+$, $^{73}C_3H_5O_2^+$, and $^{87}C_4H_7O_2^+$ peaks in a positive secondary ion spectrum of TOF-SIMS.

The presence of the 4-(aminomethyl)benzenecarboxyimidamide skeleton on the innermost surface subjected to TOF-SIMS measurement can be confirmed from at least one peak selected from the group consisting of $^{106}C_7H_8N^+$, $^{117}C_7H_5N_2^+$, $^{13}C_8H_{10}N_2^+$, and $^{148}C_8H_{10}N_3^+$ peaks in a positive secondary ion spectrum of TOF-SIMS, and a $^{119}C_7H_7N_2^-$ peak in a negative secondary ion spectrum of TOF-SIMS. The presence of the benzamidine skeleton can be confirmed from a $^{119}C_7H_7N_2^-$ peak in a negative secondary ion spectrum of TOF-SIMS. The presence of the methoxy benzene sulfonamide skeleton can be confirmed from at least one peak selected from the group consisting of a $^{117}C_7H_7SO_3^+$ peak in a positive secondary ion spectrum, and $^{64}SO_2^-$, $^{171}C_7H_7SO_3^-$, $^{186}C_7H_8SNO_3^-$, and $^{212}C_9H_{10}SNO_3^-$ peaks in a negative secondary ion spectrum.

The presence of the betaine compound on the innermost surface subjected to TOF-SIMS measurement can be confirmed from at least one peak selected from the group consisting of $^{94}CH_2SO_3^-$, $^{150}C_4H_8NSO_3^-$, and $^{166}C_5H_{12}NSO_3^-$ peaks in a negative secondary ion spectrum of TOF-SIMS.

When the cationic polymer described later is, for example, PEI, the presence of the PEI on the innermost surface can be confirmed from at least one peak selected from the group consisting of $^{18}NH_4^+$, $^{28}CH_2N^+$, $^{43}CH_3N_2^+$, and $^{70}C_4H_8N^+$ peaks in a positive secondary ion spectrum of TOF-SIMS, and $^{26}CN^-$ and $^{42}CNO^-$ peaks in a negative secondary ion spectrum of TOF-SIMS.

When the anionic polymer described later is, for example, polyacrylic acid (hereinafter referred to as "PAA"), the presence of the PAA on the innermost surface can be confirmed from a $^{71}C_3H_3O_2^-$ peak in a negative secondary ion spectrum of TOF-SIMS.

When the weft yarn used to form the tubular woven construct as a vascular prosthesis is, for example, a polyethylene terephthalate microfiber yarn, the presence of the polyethylene terephthalate can be confirmed from at least one peak selected from the group consisting of $^{16}C_6H_4^+$, $^{104}C_7H_4NO^+$, $^{105}C_7H_5O^+$, and $^{149}C_8H_5O_3^+$ peaks in a positive secondary ion spectrum of TOF-SIMS, and $^{76}C_6N_4^-$, $^{120}C_7H_4O_2^-$, $^{121}C_7H_5O_2^-$, $^{147}C_9H_7O_2^-$ and $^{165}C_8H_5O_4^-$ peaks in a negative secondary ion spectrum of TOF-SIMS.

When the anionic polymer is PAA in the present invention, there are preferred ranges for the abundance ratio of the 4-(aminomethyl)benzenecarboxyimidamide or benzamidine skeleton relative to PAA on the innermost surface, and for the abundance ratio of the methoxy benzene sulfonamide skeleton relative to PAA on the innermost surface. When the presence of PAA is confirmed from a $^{71}C_3H_3O_2^-$ peak in a negative secondary ion spectrum of TOF-SIMS, and the presence of the 4-(aminomethyl)benzenecarboxyimidamide or benzamidine skeleton is confirmed from a $^{119}C_7H_7N_2^-$ peak in a negative secondary ion spectrum of TOF-SIMS, the peak ratio $^{119}C_7H_7N_2^-/^{71}C_3H_3O_2^-$ is preferably 0.05 or more. When the presence of PAA is confirmed from a $^{71}C_3H_3O_2^-$ peak in a negative secondary ion spectrum of TOF-SIMS, and the presence of the methoxy benzene sulfonamide skeleton is confirmed from $^{64}SO_2^-$, $^{171}C_7H_7SO_3^-$ and $^{186}C_7H_8SNO_3^-$ peaks in a negative secondary ion spectrum of TOF-SIMS, the peak ratio $^{64}SO_2^-/^{71}C_3$,$H_3$,$O_2^-$ is preferably 0.6 or more, the peak ratio $^{171}C_7H_7SO_3^-/^{71}C_3H_3O_2^-$ is preferably 1.1 or more, and the peak ratio $^{186}C_7H_8SNO_3^-/^{71}C_3H_3O_2^-$ is preferably 0.5 or more.

We conducted further research in connection with the present invention to achieve both antithrombogenicity and cellular affinity with minimal elution of the antithrombogenic material B from the vascular prosthesis . As a result, we found that there is a preferred value for the abundance ratio of the C3 split peak component, which is attributed to C=O bonds and suggests the presence of carbonyl groups, relative to the C1s peak, which indicates the presence of carbon atoms on the inner surface subjected to XPS measurement.

That is, we found that the abundance ratio of the C3 split peak component to all the components of the C1s peak on the inner surface subjected to XPS measurement is preferably 1.0 atomic percent or more, more preferably 2.0 atomic percent or more, still more preferably 3.0 atomic percent or more. When the abundance ratio of the C3 split peak component to all the components of the C1s peak on the inner surface subjected to XPS measurement is 1.0 atomic percent or more, the antithrombogenic material B bound to the tubular woven construct having the blood-contacting inner surface is present in a sufficient amount, and as a result, higher and longer-lasting antithrombogenicity can be achieved as compared with cases where the antithrombogenic material is covalently bound to the tubular woven construct by irradiation as described in Patent Literature 15 and 16. When the abundance ratio of the C3 split peak component to all the components of the C1s peak on the inner surface subjected to XPS measurement is less than 1.0 atomic percent, the number of carbonyl-derived covalent amide bonds formed between the antithrombogenic material B and the warp and weft yarns that form the tubular woven construct having the blood-contacting inner surface is small, and consequently the amount of the antithrombogenic material B bound to the tubular woven construct is small, and as a result, the desired antithrombogenicity is less likely to be achieved.

We also found that, when the antithrombogenic material B is used in the vascular prosthesis, the abundance ratio of nitrogen atoms relative to all the atoms on the inner surface as determined from the N1s peak, which indicates the abundance of nitrogen atoms, as measured by XPS is preferably from 1.0 to 12.0 atomic percent, more preferably from 2.0 to 11.0 atomic percent, still more preferably from 3.0 to 10.0 atomic percent.

When the number average molecular weight of the hydrophilic polymer skeleton in the antithrombogenic material B is excessively small in the present invention, the anti-platelet adhesion activity is small, and as a result, the optimum antithrombogenicity is less likely to be exhibited immediately after implantation of the vascular prosthesis. On the other hand, when the number average molecular weight of the hydrophilic polymer skeleton is excessively large, the anti-platelet adhesion activity is high, but the moiety that exhibits anti-thrombin activity is encapsulated in the hydrophilic polymer skeleton, and as a result, again, the optimum antithrombogenicity is less likely to be achieved. Accordingly, the number average molecular weight of the hydrophilic polymer skeleton is preferably 1,500 to 20,000, more preferably 2,000 to 10,000.

The antithrombogenic material B may further contain the cationic polymer as described above. As described above, the antithrombogenic material B herein contains the following three types of skeletal structures: the hydrophilic polymer skeleton, the 4-(aminomethyl)benzenecarboxyimidamide or benzamidine skeleton, and the methoxy benzene sulfonamide skeleton, wherein the hydrophilic polymer skeleton contains, as a constituent monomer, a compound selected from the group consisting of ethylene glycol, propylene glycol, vinylpyrrolidone, vinyl alcohol, vinyl caprolactam, vinyl acetate, styrene, methyl methacrylate, hydroxyethyl methacrylate, and siloxane.

The antithrombogenic materials of the present invention, i.e., the antithrombogenic material A and the antithrombogenic material B, preferably further contain an anionic polymer containing, as a constituent monomer, a compound selected from the group consisting of acrylic acid, methacrylic acid, $\alpha$-glutamic acid, $\gamma$-glutamic acid, and aspartic acid; or an anionic compound selected from the group consisting of citric acid and dicarboxylic acids such as oxalic acid, malonic acid, succinic acid, fumaric acid, glutaric acid, adipic acid, pimelic acid, suberic acid, azelaic acid, sebacic acid, malic acid, tartaric acid, and dodecanedioic acid.

Specific examples of the anionic polymer are not particularly limited, but it is advantageous when the weight ratio of anionic functional groups in the polymer is large because a large amount of the anionic polymer can be bound to the warp and weft yarns that form the tubular woven construct having the blood-contacting inner surface, or to another antithrombogenic material. Therefore, the anionic polymer is preferably PAA, poly (methacrylic acid), poly (a-glutamic acid), poly (y-glutamic acid) or poly(aspartic acid), more preferably

PAA.

Specific examples of the PAA include "Polyacrylic Acid" (Wako Pure Chemical Industries, Ltd.). The PAA may be a copolymer with one or more other monomers or a modified PAA polymer as long as the effects of the present invention are not deteriorated.

The anionic polymer may be a copolymer with a non-anionic monomer including, but being not limited to, ethylene glycol, propylene glycol, vinylpyrrolidone, vinyl alcohol, vinyl caprolactam, vinyl acetate, styrene, methyl methacrylate, hydroxyethyl methacrylate, and siloxane, which serve as constituent monomers B. An excessively large amount of the non-anionic constituent monomer B used to form a copolymer with the anionic polymer may result in a small amount of the copolymer bound to the tubular woven construct having the blood-contacting inner surface, or to another antithrombogenic material. Thus, the amount of the non-anionic constituent monomer B is preferably 10% by weight or less.

For safety reasons etc., elution of the anionic polymer into the blood is not preferred. Thus, the anionic polymer is preferably bound to, more preferably covalently bound to, the warp and weft yarns that form the tubular woven construct having the blood-contacting inner surface.

The cationic polymer may be a homopolymer or a copolymer. When the anionic polymer is a copolymer, the copolymer may be any of a random copolymer, a block copolymer, a graft copolymer, and an alternating copolymer.

A constituent monomer of the anionic copolymer other than acrylic acid, methacrylic acid, α-glutamic acid, γ-glutamic acid or aspartic acid is not particularly limited, and may be, for example, ethylene glycol, propylene glycol, vinylpyrrolidone, vinyl alcohol, vinyl caprolactam, vinyl acetate, styrene, methyl methacrylate, hydroxyethyl methacrylate, or siloxane, which serves as a constituent monomer B. An excessively large amount of the constituent monomer B by weight may result in a small number of reaction sites for binding of the anionic copolymer to the warp and weft yarns that form the tubular woven construct having the blood-contacting inner surface, or to another antithrombogenic material. Thus, the amount by weight of the constituent monomer B based on the total weight of the anionic polymer is preferably 10% by weight or less.

The anionic compound is not particularly limited, but it is advantageous when the weight ratio of anionic functional groups in the compound is large because a large amount of the anionic compound can be bound to the tubular woven construct having the blood-contacting inner surface, or to another antithrombogenic material. Therefore, the anionic compound is preferably oxalic acid, malonic acid, succinic acid, fumaric acid, glutaric acid, adipic acid, pimelic acid, suberic acid, azelaic acid, sebacic acid, malic acid, tartaric acid or citric acid, and is more preferably succinic acid.

An excessively small weight average molecular weight of the anionic polymer may result in a small amount of the anionic polymer bound to the tubular woven construct having the blood-contacting inner surface, or to another antithrombogenic material, and consequently higher and longer-lasting antithrombogenicity is less likely to be achieved. On the other hand, an excessively large weight average molecular weight of the anionic polymer may result in encapsulation of the antithrombogenic materials in the anionic polymer. Accordingly, the weight average molecular weight of the anionic polymer is preferably 600 to 2, 000, 000, more preferably 10, 000 to 1,000,000.

The tubular woven construct of the present invention is suitable as a base material of a vascular prosthesis. The vascular prosthesis produced using the tubular woven construct has advantages of being less likely to cause leakage of blood and having both antithrombogenicity and cellular affinity. Due to these advantages, the tubular woven construct can be widely used as a base material of various types of vascular prostheses. The tubular woven construct is especially suitable as a base material of a vascular prosthesis with a small luminal diameter, and can serve as an alternative to currently available vascular prostheses with a small luminal diameter that cannot achieve good performance in the long run and cannot be applied to clinical practice. For this application, the luminal diameter of the vascular prosthesis of the present invention is preferably from 1 to 10 mm, more preferably from 1 to 6 mm.

## EXAMPLES

The present invention will be specifically described with reference to Examples, but the present invention is not limited thereto. Various alterations and modifications are possible within the technical scope of the present invention. The various properties evaluated in the Examples were measured as follows.

### Measurement methods

#### (1) Total fineness and filament fineness

The total fineness of a yarn was determined as a mass-corrected fineness in accordance with method A in JIS L 1013 (2010) 8.3.1 under a predetermined load of 0.045 cN/dtex. The total fineness was divided by the number of filaments to determine the filament fineness.

#### (2) Elongation and recovery percentage of elongation

A single strand of a yarn was mounted on a tensile testing machine with a clamp distance of 20 cm, and a load (initial load) of 0.1 g/dtex was applied. The yarn was stretched at a tensile speed of 20 cm/min to a predetermined stretch rate (a) (i.e., the yarn was stretched by a%), and the yarn was maintained in the stretched state for 1 minute. The clamp was returned to the original position so that the clamp distance was the initial distance (20 cm). The elongation (b) of the yarn under a load of zero N was determined from the S-S curve of the tensile test, and the recovery percentage of elongation was determined by the formula below. The measurement was performed three times and the mean value was calculated.

Stretch rate (%) = a (the measurement was performed at a = 10% and 20%.)

$$\text{Recovery percentage of elongation at stretch rate of a\% (\%)} = (a-b)/a \times 100$$

(3) Cover factor

The cover factor (CF) is a value calculated from the total fineness of the warp or weft yarn and the density of the warp or weft threads in the fabric. The cover factor is expressed by the following formula:

$$\text{Warp cover factor (CFa)} = Dw^{1/2} \times Nw,$$

or

$$\text{Weft cover factor (CFb)} = Df^{1/2} \times Nf,$$

wherein Dw is the total fineness (dtex) of the warp yarn, Df is the total fineness (dtex) of the weft yarn, Nw is the density of the warp threads in the fabric (ends/2.54 cm), and Nf is the density of the weft threads in the fabric (picks/2.54 cm).

The density of the threads in the fabric was determined as follows. A tubular woven construct was cut open in the longitudinal direction. The inner surface was photographed at a magnification of 50 times under a VHX-2000 microscope (KEYENCE CORPORATION), and the number of warp and weft threads per inch was counted.

(4) Kink resistance

The kink resistance was evaluated by measuring the kink radius in accordance with the guidance of ISO 7198. Briefly, a tubular woven construct was formed into a loop, and the radius of the loop was gradually decreased until apparent kinking occurred. A cylindrical mandrel with a known radius was placed in the loop to measure the radius. In the test, internal pressure was not applied for the purpose of the evaluation of the genuine kink resistance of the tubular woven construct.

(5) Width of tubular woven construct

A tubular woven construct was flattened by pressing it with a metal ruler placed perpendicular to the longitudinal direction of the woven construct, and the width of the woven construct was measured. The measurement was performed on randomly selected three positions on the tubular woven construct. Each position was measured once and the mean value of the three measurements was calculated.

(6) Tensile test

Strength at break, elongation at break, and elongation per mm in width of tubular woven construct under a load of 3.3 N

The measurement was performed by the cut strip test method in accordance with method A in JIS L 1096.

A strip of a tubular woven construct was mounted on a tensile testing machine at a clamp distance of 10 cm, and the measurement was carried out at a tensile speed of 20 cm/min. From the S-S curve of the tensile test, the elongation per mm in width of the tubular woven construct under a load of 3.3 N, the load at break, and the elongation at break of the specimen were determined.

(7) Water permeability

A multi-layer tubular woven construct was closed at one end. From the other end, water at 25°C as sufficiently clean as tap water was fed into the woven construct for 20 minutes under the condition that the hydraulic pressure applied to the inner wall was 120 mmHg (16 kPa). Then, the amount of the water that leaked through the wall of the tubular woven construct per minute was measured. The measured amount was divided by the surface area ($cm^2$) of the multi-layer tubular woven construct. The obtained value was taken as the water permeability. The measurement was performed three times and the mean value was calculated.

(8) Leakage of blood

A tubular woven construct was closed at one end, and the other end was connected to a tube and other devices for feeding bovine blood at 25°C. The bovine blood was fed into the tubular woven construct for 20 minutes under the condition that the pressure applied to the inner wall of the woven construct was 120 mmHg (16 kPa) so that the blood infiltrated from the inner surface to the outer surface of the woven construct, until the whole vascular prosthesis was fully impregnated with the blood. Then, the blood permeating through the woven construct was collected for 5 to 20 minutes. The amount (mL) of the collected blood was divided by the inner surface area ($cm^2$) of the woven construct and unit time (min). The obtained value was taken as the amount of the leakage of the blood at 120 mmHg (16 kPa) . The measurement was performed three times and the mean value was calculated.

(9) Thickness analysis of antithrombogenic material layer with STEM

The thickness of an antithrombogenic material layer was determined with a STEM. Cross-sectional samples of a vascular prosthesis were prepared by ultramicrotomy. The thickness where sulfur atoms derived from the antithrombogenic material were observed was measured by STEM-EDX. The thickness where nitrogen atoms derived from the antithrombogenic material were observed was measured by STEM-EELS. The STEM analysis was performed under the conditions below. The thickness was measured at at least three randomly selected positions and the measured values were averaged to determine the mean thickness.

Measurement conditions

Apparatus: field emission transmission electron microscope JEM-2100F (JEOL Ltd.)
EELS detector: GIF Tridiem (GATAN, Inc.)
EDX detector: JED-2300T (JEOL Ltd.)
Image acquisition: Digital Micrograph (GATAN, Inc.)
Sample preparation: ultramicrotomy (the samples were embedded in an acrylic resin, and the sliced sections were placed on a copper microgrid.)
Accelerating voltage: 200 kV
Beam diameter: 0.7 nm
Energy resolution: about 1.0 eVFWHM

(10) Assessment of antithrombogenicity of vascular prosthesis implanted in the carotid arteries of dogs

A vascular prosthesis was implanted in the carotid arteries of dogs with reference to P. C. Begovac et al. (Eur Vasc Endovasc Surg 25, 432-437, 2003) etc. The implanted vascular prosthesis and the native blood vessel to which the vascular prosthesis was anastomosed were observed by vascular ultrasound and angiography at regular intervals to examine the presence or absence of thrombi and clogging of the vascular prosthesis. The vascular prosthesis was judged to have high antithrombogenicity and scored as "good" when no complete clogging of the vascular prosthesis was observed 28 days after implantation, and the vascular prosthesis was judged to have insufficient antithrombogenicity and scored as "poor" when complete clogging of the vascular prosthesis was observed 28 days after implantation.

(11) Assessment of cellular affinity of vascular prosthesis implanted in the carotid arteries of dogs

A vascular prosthesis was implanted in the carotid arteries of dogs in the same manner as in the above assessment 2. The vascular prosthesis was extracted 28 days after implantation, and the specimen was HE stained. The stained specimen was observed under a microscope, and the distance was measured from the anastomosis site between the vascular prosthesis and the native blood vessel to the end of the region where vascular endothelial cells settled. The vascular prosthesis was judged to have very high cellular affinity and scored as "very good" when the length of the region where vascular endothelial cells settled was 5.0 mm or longer. The vascular prosthesis was judged to have high cellular affinity and scored as "good" when the length of the region where vascular endothelial cells settled was from 2.0 mm or longer but shorter than 5.0 mm. The vascular prosthesis was judged to have insufficient cellular affinity and scored as "poor" when the length of the region where vascular endothelial cells settled was shorter than 2.0 mm.

Example 1

A false twisted multifilament yarn (elastic fiber yarn) of 24 filaments having a filament fineness of about 2.33 dtex and a total fineness of 56 dtex, the filaments being composite cross-section fiber filaments with a cross section of side-by-side arrangement of PET and PPT (PET/PPT "bi-metallic" type DTY yarn, the recovery percentage of elongation at a stretch rate of 20% was 45%, and the recovery percentage of elongation at a stretch rate of 10% was 60%) was used as a warp yarn to form the inner and outer layers of a tubular woven fabric. A false twisted PET microfiber yarn of 144 filaments having a filament fineness of about 0.31 dtex and a total fineness of 44 dtex was used as a weft yarn to form the inner layer of the tubular woven fabric. A PET monofilament yarn having a total fineness of 180 dtex (having a thickness of 130 μm (as measured on a photograph of the surface of the filament taken at a magnification of 400 times under a VHX-2000 microscope (KEYENCE CORPORATION)) was used as a weft yarn to form the outer layer.

The two sets of warp yarns and the two sets of weft yarns were interwoven in plain double-weave on a shuttle loom set at 202 picks per cm to form a double-weave tubular woven fabric of 3 mm in luminal diameter. The tubular woven fabric was scoured at 80°C, treated in boiling water for 5 minutes, and dry-heated at 120°C. Into the fabric, a rod mandrel was inserted and the fabric was heat-set at 170°C. The thus produced tubular woven construct was subjected to the assessment of kink resistance, tensile properties, cover factor, water permeability, and leakage of blood. The results are shown in Tables 1, 2 and 3. The results of the tensile properties indicated that the tubular woven construct had flexibility and stretchability as well as excellent shape-retaining properties. The tubular woven construct also had the kink resistance, the water permeability and the blood impermeability that are required for a vascular prosthesis.

Example 2

A tubular woven fabric was produced in the same manner as in Example 1, except that the weft yarn used to form the inner layer was a PET microfiber yarn of 630 filaments having a filament fineness of about 0.08 dtex and a total fineness of 52.8 dtex and that the number of picks per cm was set at 186.

The produced woven fabric was subjected to the assessment of kink resistance, tensile properties, cover factor, water permeability, and leakage of blood. The results are shown in Tables 1, 2 and 3. The results of the tensile properties indicated that the tubular woven construct had flexibility and stretchability as well as excellent shape-retaining properties. The tubular woven construct also had the kink resistance, the water permeability and the blood impermeability that are required for a vascular prosthesis.

Example 3

A tubular woven fabric was produced in the same manner as in Example 1, except that the number of picks per cm was changed from 202 to 158.

The produced woven fabric was subjected to the assessment of kink resistance, tensile properties, cover factor, water permeability, and leakage of blood. The results are shown in Tables 1, 2 and 3. The elongation per m in width of the tubular woven construct under a load of 3.3 N was higher than those of Examples 1 and 2, and thus the tubular woven construct had flexibility and stretchability. The tubular woven construct also had the kink resistance, the water permeability and the blood impermeability that are required for a vascular prosthesis.

Example 4

A tubular woven fabric was produced in the same manner as in Example 2, except that the number of picks per cm was changed from 186 to 125.

The produced woven fabric was subjected to the assessment of kink resistance, tensile properties, cover factor, water permeability, and leakage of blood. The results are shown in Tables 1, 2 and 3. The results of the tensile properties indicated that the tubular woven construct had similar flexibility and stretchability to those of Example 3. The tubular woven construct also had the kink resistance, the water permeability and the blood impermeability that are required for a vascular prosthesis.

Example 5

The tubular woven construct of Example 1 was immersed in an aqueous solution containing 5.0 wt% potassium permanganate (Wako Pure Chemical Industries, Ltd.) and 0.6 mol/L sulfuric acid (Wako Pure Chemical Industries, Ltd.) at 60°C for 3 hours to allow hydrolysis and oxidation reaction to proceed.

The tubular woven construct was then immersed in an aqueous solution containing 0.5 wt% DMT-MM (4-(4,6-dimethoxy-1,3,5-triazin-2-yl)-4-methylmorpholinium chloride n-hydrate; Wako Pure Chemical Industries, Ltd.) and 5.0

wt% PEI (LUPASOL (registered trademark) P; BASF) at 30°C for 2 hours to allow PEI to covalently bind to the tubular woven construct via condensation reaction.

The tubular woven construct was then immersed in an aqueous solution of ethyl bromide (Wako Pure Chemical Industries, Ltd.) in 1 wt% methanol at 35°C for 1 hour. The solution was then heated to 50°C, and the reaction was continued for 4 hours to allow the formation of quaternary ammonium salts of the PEI covalently bound to the tubular woven construct.

Finally, the tubular woven construct was immersed in an aqueous solution containing 0.75 wt% heparin sodium (Organon API) and 0.1 mol/L sodium chloride (pH = 4) at 70°C for 6 hours to allow heparin to ionically bind to the quaternary ammoniated PEI. Thus a vascular prosthesis on which an antithrombogenic material layer was formed (sample 1) was obtained.

The obtained vascular prosthesis (sample 1) was subjected to the assessment of antithrombogenicity and cellular affinity by implantation test of the vascular prosthesis into the carotid arteries of dogs. The results are shown in Table 4. As shown in Table 4, in the antithrombogenicity assessment, no complete clogging was observed 28 days after implantation and the vascular prosthesis was scored as "good". In the cellular affinity assessment, the length of the region where vascular endothelial cells settled was 5.0 mm or longer and the vascular prosthesis was scored as "very good".

## Example 6

In the same manner as in Example 5, the tubular woven construct of Example 1 was subjected to hydrolysis and oxidation followed by covalent bonding of PEI via condensation reaction, and then immersed in a solution of 0.5 wt% DMT-MM and 40 wt% succinic anhydride (Wako Pure Chemical Industries, Ltd.) in dimethylacetamide at 50°C for 17 hours to allow the reaction to proceed.

The tubular woven construct was then immersed in an aqueous solution of 0.5 wt% DMT-MM and 5.0 wt% PEI at 30°C for 2 hours to allow the reaction to proceed. In the same manner as in Example 5, the tubular woven construct was treated with ethyl bromide to allow the formation of quaternary ammonium salts of the PEI, and was then treated with heparin sodium. Thus a vascular prosthesis on which an antithrombogenic material layer was formed (sample 2) was obtained.

The obtained vascular prosthesis (sample 2) was subjected to the assessment of antithrombogenicity and cellular affinity by implantation test of the vascular prosthesis into the carotid arteries of dogs. The results are shown in Table 4. As shown in Table 4, in the antithrombogenicity assessment, no complete clogging was observed 28 days after implantation and the vascular prosthesis was scored as "good". In the cellular affinity assessment, the length of the region where vascular endothelial cells settled was 2.0 mm or longer but shorter than 5.0 mm and the vascular prosthesis was scored as "good".

## Example 7

In the same manner as in Example 5, the tubular woven construct of Example 1 was subjected to hydrolysis and oxidation followed by covalent bonding of PEI via condensation reaction, and then immersed in an aqueous solution of 0.5 wt% DMT-MM and 0.5 wt% PAA (polyacrylic acid, with a weight average molecular weight of 1,000,000; Wako Pure Chemical Industries, Ltd.) at 30°C for 2 hours to allow the reaction to proceed.

The tubular woven construct was then immersed in an aqueous solution of 0.5 wt% DMT-MM and 5.0 wt% PEI at 30°C for 2 hours to allow the reaction to proceed. In the same manner as in Example 5, the tubular woven construct was treated with ethyl bromide to allow the formation of quaternary ammonium salts of the PEI, and was then treated with heparin sodium. Thus a vascular prosthesis on which an antithrombogenic material layer was formed (sample 3) was obtained.

The obtained vascular prosthesis (sample 3) was subjected to the assessment of antithrombogenicity and cellular affinity by implantation test of the vascular prosthesis into the carotid arteries of dogs. The results are shown in Table 4. As shown in Table 4, in the antithrombogenicity assessment, no complete clogging was observed 28 days after implantation and the vascular prosthesis was scored as "good". In the cellular affinity assessment, the length of the region where vascular endothelial cells settled was 2.0 mm or longer but shorter than 5.0 mm and the vascular prosthesis was scored as "good".

## Example 8

A vascular prosthesis was produced in the same manner as in Example 7, except that poly(allylamine hydrochloride) (hereinafter referred to as "PAH") (with a weight average molecular weight of 900,000; Sigma-Aldrich) was used in place of the PEI. Another vascular prosthesis was produced in the same manner as in Example 7, except that

poly-L-lysine hydrobromide (hereinafter referred to as "PLys") (with a weight average molecular weight of 30,000 to 70,000; Sigma-Aldrich) was used in place of the PEI.

The vascular prosthesis having an antithrombogenic material layer formed using PAH was designated as sample 4, and the vascular prosthesis having an antithrombogenic material layer formed using PLys was designated as sample 5.

The obtained vascular prostheses (samples 4 and 5) were subjected to the assessment of antithrombogenicity and cellular affinity by implantation test of the vascular prostheses into the carotid arteries of dogs. The results are shown in Table 4. As shown in Table 4, in the antithrombogenicity assessment, no complete clogging was observed 28 days after implantation and the vascular prostheses were scored as "good". In the cellular affinity assessment, the length of the region where vascular endothelial cells settled was 2.0 mm or longer but shorter than 5.0 mm and the vascular prostheses were scored as "good".

## Example 9

A vascular prosthesis (sample 6) was produced in the same manner as in Example 5, except that dextran sulfate sodium (Wako Pure Chemical Industries, Ltd.) was used in place of heparin sodium to form an antithrombogenic material layer.

The obtained vascular prosthesis (sample 6) was subjected to the assessment of antithrombogenicity and cellular affinity by implantation test of the vascular prosthesis into the carotid arteries of dogs. The results are shown in Table 4. As shown in Table 4, in the antithrombogenicity assessment, no complete clogging was observed 28 days after implantation and the vascular prosthesis was scored as "good". In the cellular affinity assessment, the length of the region where vascular endothelial cells settled was 2.0 mm or longer but shorter than 5.0 mm and the vascular prosthesis was scored as "good".

## Example 10

In the same manner as in Example 5, the tubular woven construct of Example 1 was subjected to hydrolysis and oxidation, and then immersed in an aqueous solution containing 1.0 wt% of compound A (general formula (X) below), sodium hydroxide in an amount of 2 molar equivalents relative to compound A and DMT-MM in an amount of 3 molar equivalents relative to compound A at 30°C for 2 hours to allow compound A to covalently bind to the tubular woven fabric 1 via condensation reaction. Thus a vascular prosthesis on which an antithrombogenic material layer was formed (sample 7) was obtained.

(X)

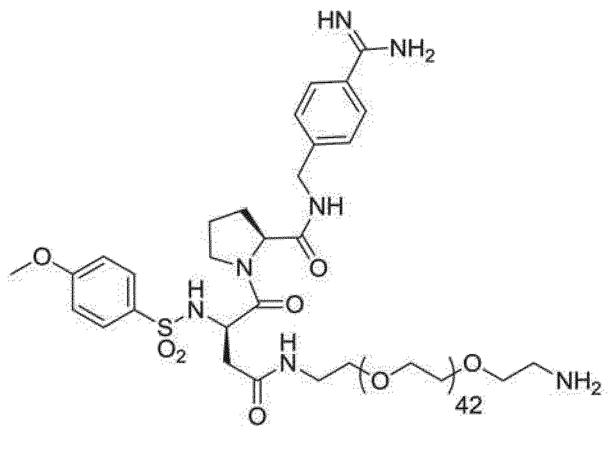

The obtained vascular prosthesis (sample 7) was subjected to the assessment of antithrombogenicity and cellular affinity by implantation test of the vascular prosthesis into the carotid arteries of dogs. The results are shown in Table 4. As shown in Table 4, in the antithrombogenicity assessment, no complete clogging was observed 28 days after implantation and the vascular prosthesis was scored as "good". In the cellular affinity assessment, the length of the region where vascular endothelial cells settled was 2.0 mm or longer but shorter than 5.0 mm and the vascular prosthesis was scored as "good".

Example 11

Vascular prostheses were produced in the same manner as in Example 10, except that compound B (general formula (XI) below), compound C (general formula (XII) below) or compound D (general formula (XIII) below) was used in place of compound A.

(XI)

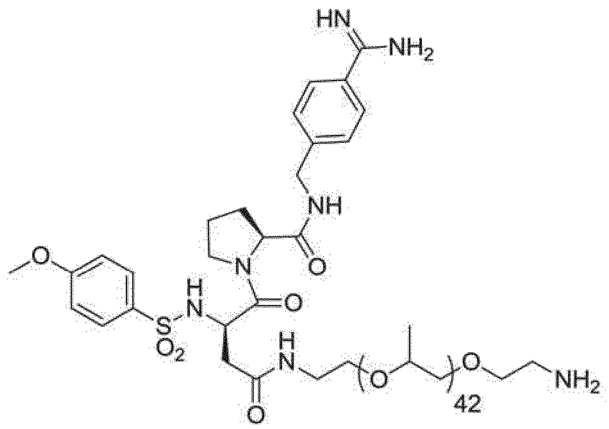

(XII)

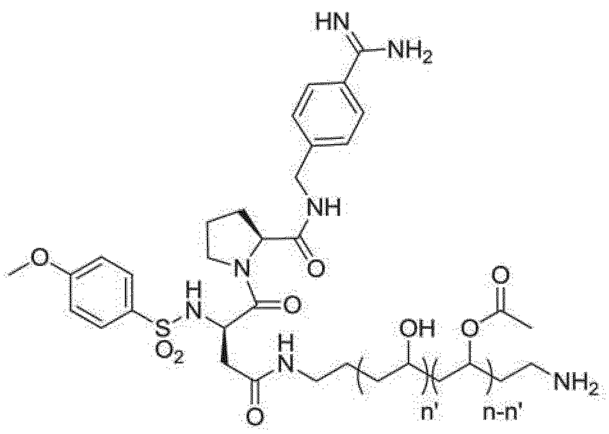

(In the formula, n is 42, and the saponification degree (n'/n × 100) is 85 to 90%.)

(XIII)

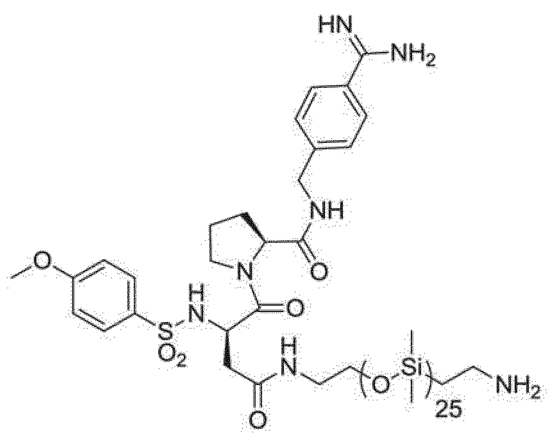

The vascular prosthesis having an antithrombogenic material layer formed using compound B was designated as sample 8. The vascular prosthesis having an antithrombogenic material layer formed using compound C was designated as sample 9. The vascular prosthesis having an antithrombogenic material layer formed using compound D was designated as sample 10.

The obtained vascular prostheses (samples 8 to 10) were subjected to the assessment of antithrombogenicity and cellular affinity by implantation test of the vascular prostheses into the carotid arteries of dogs. The results are shown in Table 4. As shown in Table 4, in the antithrombogenicity assessment, no complete clogging was observed 28 days after implantation and the vascular prostheses were scored as "good". In the cellular affinity assessment, the length of the region where vascular endothelial cells settled was 2.0 mm or longer but shorter than 5.0 mm and the vascular prostheses were scored as "good".

Example 12

In the same manner as in Example 5, the tubular woven construct of Example 1 was subjected to hydrolysis and oxidation followed by covalent bonding of PEI via condensation reaction, and then immersed in an aqueous solution of 0.5 wt% DMT-MM and 0.5 wt% PAA (with a weight average molecular weight of 1,000,000; Wako Pure Chemical Industries, Ltd.) at 30°C for 2 hours to allow the reaction to proceed.

The tubular woven construct was then immersed in an aqueous solution containing 1.0 wt% of compound A, sodium hydroxide in an amount of 2 molar equivalents relative to compound A and DMT-MM in an amount of 3 molar equivalents relative to compound A at 30°C for 2 hours to allow compound A to covalently bind to the tubular woven construct via condensation reaction. Thus a vascular prosthesis on which an antithrombogenic material layer was formed (sample 11) was obtained.

The obtained vascular prosthesis (sample 11) was subjected to the assessment of antithrombogenicity and cellular affinity by implantation test of the vascular prosthesis into the carotid arteries of dogs. The results are shown in Table 4. As shown in Table 4, in the antithrombogenicity assessment, no complete clogging was observed 28 days after implantation and the vascular prosthesis was scored as "good". In the cellular affinity assessment, the length of the region where vascular endothelial cells settled was 2.0 mm or longer but shorter than 5.0 mm and the vascular prosthesis was scored as "good".

Comparative Example 1

A tubular woven construct was produced in the same manner as in Example 1, except that the warp and weft yarns used to form the inner layer of the tubular woven fabric were a PET microfiber drawn yarn of 144 filaments having a filament fineness of about 0.31 dtex and a total fineness of 44 dtex (the recovery percentage of elongation at a stretch rate of 20% was 25%, and the recovery percentage of elongation at a stretch rate of 10% was 40%), that the warp yarn used to form the outer layer was a false twisted PET multifilament yarn of 24 filaments having a filament fineness of about 2.33 dtex and a total fineness of 56 dtex (the recovery percentage of elongation at a stretch rate of 20% was 25%, and the recovery percentage of elongation at a stretch rate of 10% was 40%), that the weft yarn used to form the outer layer was a PET monofilament yarn having a total fineness of 180 dtex (having a thickness of 130 μm (as measured on a photograph of the surface of the filament taken at a magnification of 400 times under a VHX-2000 microscope (KEYENCE CORPORATION)), and that the number of picks per cm was set at 240.

The produced tubular woven construct was subjected to the assessment of kink resistance, tensile properties, cover factor, water permeability, and leakage of blood. The results are shown in Tables 1, 2 and 3. The tubular woven construct had the kink resistance, the water permeability and the blood impermeability that are required for a vascular prosthesis, but the tubular woven construct was excessively rigid and had no stretchability.

Comparative Example 2

A tubular woven fabric was produced in the same manner as in Comparative Example 1, except that the warp and weft yarns used to form the inner layer of the tubular woven fabric were a false twisted PET microfiber yarn of 144 filaments having a filament fineness of about 0.31 dtex and a total fineness of 44 dtex (the recovery percentage of elongation at a stretch rate of 20% was 25%, and the recovery percentage of elongation at a stretch rate of 10% was 40%), and that the number of picks per cm was set at 167.

The produced tubular woven fabric was subjected to the assessment of kink resistance, tensile properties, cover factor, water permeability, and leakage of blood. The results are shown in Tables 1, 2 and 3. The tubular woven construct had the kink resistance, the water permeability and the blood impermeability that are required for a vascular prosthesis, but the tubular woven construct was excessively rigid and had no stretchability.

Comparative Example 3

A tubular woven fabric was produced in the same manner as in Example 1, except that the weft yarn used to form the inner layer of the tubular woven fabric was a false twisted PET/PPT "bi-metallic" type composite multifilament yarn of 24 filaments having a filament fineness of about 2.33 dtex and a total fineness of 56 dtex (the same yarn as used as the warp yarn in Example 1), and that the number of picks per cm was set at 220.

The produced tubular woven fabric was subjected to the assessment of kink resistance, tensile properties, cover factor, water permeability, and leakage of blood. The results are shown in Tables 1, 2 and 3. The elongation per mm in width of the tubular woven construct under a load of 3.3 N was smaller that of Example 1, and the tubular woven fabric had poor stretchability.

Comparative Example 4

A tubular woven fabric was produced in the same manner as in Comparative Example 3, except that the number of picks per cm was changed from 220 to 135.

The produced tubular woven fabric was subjected to the assessment of kink resistance, tensile properties, cover factor, water permeability, and leakage of blood. The results are shown in Tables 1, 2 and 3. The tubular woven fabric had flexibility, but the elongation at break was excessively high and the tubular woven fabric had poor shape-retaining properties.

Reference Example 1

A multi-layer tubular woven construct was produced in the same manner as in Example 1 and used as a vascular prosthesis having no antithrombogenic material layer (sample 12).

The obtained vascular prosthesis (sample 12) was subjected to the assessment of antithrombogenicity and cellular affinity by implantation test of the vascular prosthesis into the carotid arteries of dogs. The results are shown in Table 4. As shown in Table 4, in the antithrombogenicity assessment, complete clogging was observed before 28 days passed after implantation and the vascular prosthesis was scored as "poor". In the cellular affinity assessment, the length of the region where vascular endothelial cells settled was shorter than 2.0 mm and the vascular prosthesis was scored as "poor".

Table 1

| | Inner layer | | | | Outer layer | | | |
|---|---|---|---|---|---|---|---|---|
| | Warp yarn | | | | Warp yarn | | | |
| | Total fineness | Filament fineness | Density | Cover factor | Total fineness | Filament fineness | Density | Cover factor |
| | (dtex) | (dtex) | ends/ inch | Cfa1 | (dtex) | (dtex) | ends/ inch | Cfa2 |
| Example 1 | 56 | 2.33 | 265 | 1980 | 56 | 2.33 | 131 | 982 |
| Example 2 | 56 | 2.33 | 265 | 1980 | 56 | 2.33 | 131 | 982 |
| Example 3 | 56 | 2.33 | 265 | 1980 | 56 | 2.33 | 131 | 982 |
| Example 4 | 56 | 2.33 | 265 | 1980 | 56 | 2.33 | 131 | 982 |
| Comparative Example 1 | 44 | 0.31 | 265 | 1755 | 56 | 2.33 | 131 | 982 |
| Comparative Example 2 | 44 | 0.31 | 263 | 1741 | 56 | 2.33 | 133 | 998 |
| Comparative Example 3 | 56 | 2.33 | 265 | 1980 | 56 | 2.33 | 131 | 982 |
| Comparative Example 4 | 56 | 2.33 | 265 | 1980 | 56 | 2.33 | 131 | 982 |

Table 2

| | Inner layer | | | | Outer layer | | |
|---|---|---|---|---|---|---|---|
| | Weft yarn | | | | Weft yarn | | |
| | Total fineness | Filament fineness | Density | Cover factor | Fineness | Density | Cover factor |
| | (dtex) | (dtex) | picks/ inch | Cfb1 | (dtex) | picks/ inch | Cfb2 |
| Example 1 | 44 | 0.31 | 394 | 2614 | 180 | 25 | 335 |
| Example 2 | 52.8 | 0.08 | 365 | 2652 | 180 | 23 | 309 |
| Example 3 | 44 | 0.31 | 189 | 1258 | 180 | 12 | 158 |
| Example 4 | 52.8 | 0.08 | 270 | 1961 | 180 | 16 | 215 |
| Comparative Example 1 | 44 | 0.31 | 492 | 3575 | 180 | 31 | 416 |
| Comparative Example 2 | 44 | 0.31 | 281 | 1863 | 180 | 16 | 215 |
| Comparative Example 3 | 56 | 2.33 | 492 | 3681 | 180 | 31 | 416 |
| Comparative Example 4 | 56 | 2.33 | 362 | 2709 | 180 | 23 | 309 |

Table 3

| | Width of woven construct in warp direction (mm) | Tensile properties | | | Water permeability | Leakage of blood | | | Kink resistance |
|---|---|---|---|---|---|---|---|---|---|
| | | Strengthat break | Elongation at break | Elongation per mm in width of tubular woven construct under load of 3.3 N | | mL/min·120 mmHg (16 kPa)·$cm^2$ | | | (Kink radius) |
| | | N | (%) | (%) | mL/min·120 mmHg (16 kPa)·$cm^2$ | 0 min | 10 min | 20 min | (mm) |
| Example 1 | 6 | 259 | 39.8 | 4.4 | 137 | 0.13 | 0.12 | 0.11 | 17 |
| Example 2 | 6 | 208 | 37.1 | 4.7 | 136 | 0.56 | 1.15 | 0.77 | 15 |
| Example 3 | 6 | 253 | 49.7 | 10.0 | 163 | 0.11 | 0.37 | 0.56 | 13 |
| Example 4 | 6 | 193 | 40.8 | 9.1 | 161 | 2.00 | 3.49 | 1.96 | 10 |
| Comparative Example 1 | 6 | 255 | 31.0 | 1.5 | 40 | 1.10 | 0.63 | 0.49 | 26 |
| Comparative Example 2 | 6 | 239 | 27.5 | 3.1 | 62 | 0.11 | 0.07 | 0.08 | 15 |
| Comparative Example 3 | 6 | 372 | 38.5 | 2.2 | 135 | 2.52 | 1.67 | 0.95 | 20 |
| Comparative Example 4 | 6 | 259 | 55.8 | 3.9 | 166 | 4.19 | 2.91 | 1.47 | 17 |

Table 4

| | Sample | Abundance ratio of sulfur atoms (atomic percent) | Abundance ratio of nitrogen atoms (atomic percent) | Thickness of antithrombogenic material layer (nm) | Antithrombo-genicity | Cellular affinity |
|---|---|---|---|---|---|---|
| Example 5 | 1 | 3.8 | 8.3 | 66 | Good | Very good |
| Example 6 | 2 | 3.5 | 8.2 | 517 | Good | Good |
| Example 7 | 3 | 3.9 | 9.9 | 593 | Good | Good |
| Example 8 | 4 | 3.1 | 9.0 | 488 | Good | Good |
| | 5 | 3.0 | 9.1 | 498 | Good | Good |
| Example 9 | 6 | 3.5 | 8.1 | 63 | Good | Very good |
| Example 10 | 7 | - | - | 47 | Good | Good |
| Example 11 | 8 | - | - | 40 | Good | Good |
| | 9 | - | - | 44 | Good | Good |
| | 10 | - | - | 42 | Good | Good |
| Example 12 | 11 | - | - | 492 | Good | Good |
| Reference Example 1 | 12 | - | - | 0 | Poor | Poor |

INDUSTRIAL APPLICABILITY

The tubular woven construct of the present invention is suitable as a hose for transporting a fluid or a powder or for protecting linear bodies such as wires, cables and conduits, as a tubular filter, or as a base material of a vascular prosthesis.

## Claims

1. A tubular woven construct in a tubular configuration woven by interlacing warp and weft yarns, the warp yarn containing at least in part an elastic fiber yarn having a filament fineness of 1.0 dtex or more, the weft yarn containing at least in part a microfiber yarn having a filament fineness of less than 1.0 dtex.

2. The tubular woven construct according to claim 1, which satisfies the following formula: $Cfa < Cfb$, wherein Cfa is a warp cover factor and Cfb is a weft cover factor.

3. The tubular woven construct according to claim 1 or 2, which has an elongation of 4% or more in the warp direction per mm in width of the tubular woven construct under a load of 3.3 N, and has an elongation at break of 50% or less.

4. The tubular woven construct according to any one of claims 1 to 3, wherein the elastic fiber yarn having a filament fineness of 1.0 dtex or more contains composite cross-section fiber filaments formed of two types of polymers having different thermal shrinkage properties.

5. The tubular woven construct according to claim 4, wherein the two types of polymers having different thermal shrinkage properties are polyethylene terephthalate and polytrimethylene terephthalate.

6. The tubular woven construct according to any one of claims 1 to 5, which contains two or more layers.

7. The tubular woven construct according to claim 6, wherein a layer other than an innermost layer comprises a weft yarn containing at least in part a monofilament yarn having a thickness of 20 μm or more.

8. The tubular woven construct according to any one of claims 1 to 7, whose inner surface has a water permeability of 500 mL/min·120 mmHg (16 kPa) ·$cm^2$ or less.

9. A vascular prosthesis containing the tubular woven construct according to any one of claims 1 to 8 as a base material.

10. The vascular prosthesis according to claim 9, which has an antithrombogenic material layer formed by binding of an antithrombogenic material to an inner surface of the tubular woven construct to be in contact with blood, wherein the antithrombogenic material layer has a thickness of 1 to 600 nm.

11. The vascular prosthesis according to claim 10, wherein the antithrombogenic material contains a sulfur-containing anionic compound having anticoagulant activity.

12. The vascular prosthesis according to claim 10 or 11, whose inner surface, when subjected to X-ray photoelectron spectroscopy (XPS), shows an abundance ratio of sulfur atoms of 3.0 to 6.0 atomic percent relative to all the atoms on the inner surface.

13. The vascular prosthesis according to any one of claims 10 to 12, whose inner surface, when subjected to X-ray photoelectron spectroscopy (XPS), shows an abundance ratio of nitrogen atoms of 6.0 to 12.0 atomic percent relative to all the atoms on the inner surface.

14. The vascular prosthesis according to any one of claims 10 to 13, wherein the antithrombogenic material contains a cationic polymer containing, as a constituent monomer, a compound selected from the group consisting of alkyleneimines, vinyl amines, allylamine, lysine, protamines, and diallyl dimethyl ammonium chloride, and wherein the cationic polymer is covalently bound to warp and weft yarns that form the tubular woven construct.

15. The vascular prosthesis according to claim 10, wherein the antithrombogenic material is a compound containing three types of skeletal structures, wherein the three types of skeletal structures are a hydrophilic polymer skeleton, a 4-(aminomethyl)benzenecarboxyimidamide or benzamidine skeleton, and a methoxy benzene sulfonamide skeleton, wherein the hydrophilic polymer skeleton contains, as a constituent monomer, a compound selected from the group consisting of ethylene glycol, propylene glycol, vinylpyrrolidone, vinyl alcohol, vinyl caprolactam, vinyl acetate, styrene, methyl methacrylate, hydroxyethyl methacrylate, and siloxane, and wherein the compound containing the three types of skeletal structures is covalently bound to warp and weft yarns that form the tubular woven construct.

16. The vascular prosthesis according to claim 15, wherein the compound containing the three types of skeletal structures is a compound represented by any of the following general formulae (I) to (IV):

(I)

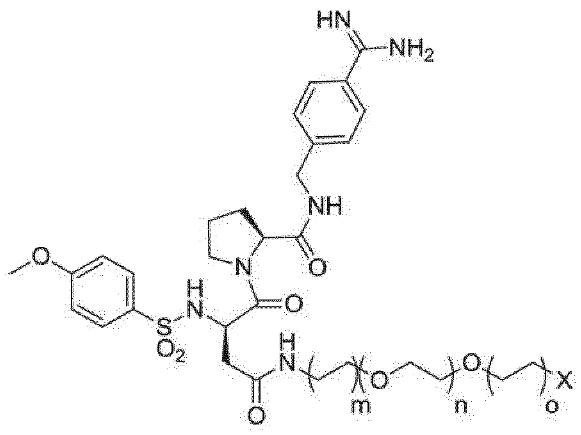

,

(II)

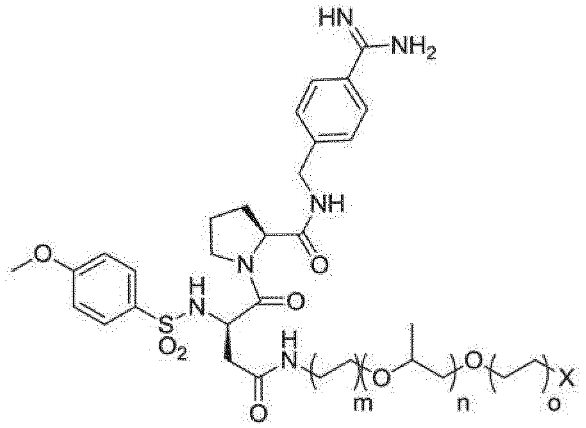

,

(III)

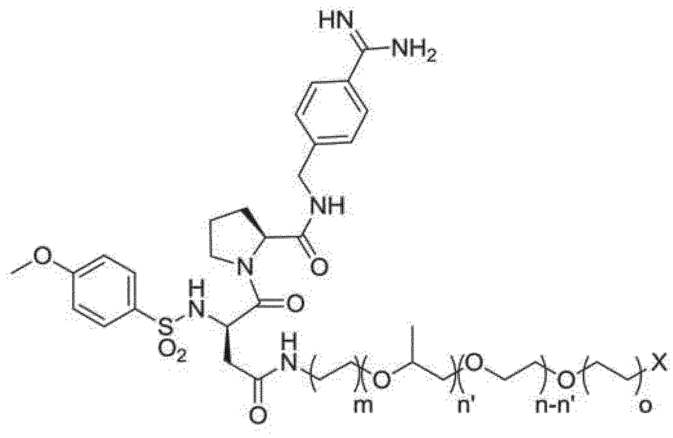

,

and

(IV)

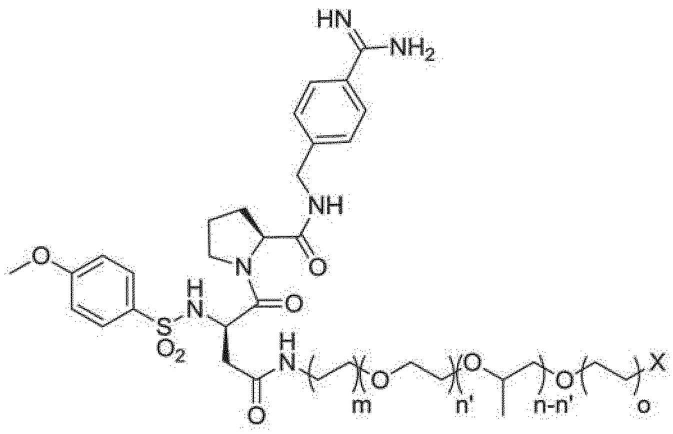

,

wherein m and o each represent an integer of 0 to 4; n represents an integer of 3 to 1000, and n' represents an integer of 3 to 1000, with the proviso that n and n' satisfy the formula: $n \geq n'$ ; and X represents a functional group selected from the group consisting of hydroxyl, thiol, amino, carboxyl, aldehyde, isocyanate, and thioisocyanate groups.

**17.** The vascular prosthesis according to any one of claims 10 to 16, wherein the antithrombogenic material contains an anionic polymer containing, as a constituent monomer, a compound selected from the group consisting of acrylic acid, methacrylic acid, α-glutamic acid, γ-glutamic acid and aspartic acid; or an anionic compound selected from the group consisting of oxalic acid, malonic acid, succinic acid, fumaric acid, glutaric acid, adipic acid, pimelic acid, suberic acid, azelaic acid, sebacic acid, malic acid, tartaric acid, and citric acid.

**INTERNATIONAL SEARCH REPORT**

International application No.
PCT/JP2016/064811

A. CLASSIFICATION OF SUBJECT MATTER
*A61L27/00*(2006.01)i, *A61F2/06*(2013.01)i, *A61L33/00*(2006.01)i, *D03D3/02*(2006.01)i, *D03D15/00*(2006.01)i

According to International Patent Classification (IPC) or to both national classification and IPC

B. FIELDS SEARCHED

Minimum documentation searched (classification system followed by classification symbols)
A61L27/00, A61F2/06, A61L33/00, D03D3/02, D03D15/00

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2016 |
| Kokai Jitsuyo Shinan Koho | 1971-2016 | Toroku Jitsuyo Shinan Koho | 1994-2016 |

Electronic data base consulted during the international search (name of data base and, where practicable, search terms used)
CAplus/MEDLINE/EMBASE/BIOSIS(STN), JSTPlus/JMEDPlus/JST7580(JDreamIII)

C. DOCUMENTS CONSIDERED TO BE RELEVANT

| Category* | Citation of document, with indication, where appropriate, of the relevant passages | Relevant to claim No. |
|---|---|---|
| Y | WO 2014/038219 A1 (Nicem, Ltd.), 13 March 2014 (13.03.2014), example 1 & US 2015/0224232 A1 example 1 & EP 2893941 A1 | 1-17 |
| Y | JP 08-080342 A (Seiren Co., Ltd.), 26 March 1996 (26.03.1996), claims 1 to 7; paragraphs [0005], [0020], [0030] (Family: none) | 1-17 |

☒ Further documents are listed in the continuation of Box C.

☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed

"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 13 June 2016 (13.06.16) | 21 June 2016 (21.06.16) |

| Name and mailing address of the ISA/ | Authorized officer |
|---|---|
| Japan Patent Office<br>3-4-3,Kasumigaseki,Chiyoda-ku,<br>Tokyo 100-8915,Japan | Telephone No. |

Form PCT/ISA/210 (second sheet) (January 2015)

**INTERNATIONAL SEARCH REPORT**

International application No.
PCT/JP2016/064811

C (Continuation). DOCUMENTS CONSIDERED TO BE RELEVANT

| Category* | Citation of document, with indication, where appropriate, of the relevant passages | Relevant to claim No. |
|---|---|---|
| Y | JP 60-99258 A (Toray Industries, Inc.), 03 June 1985 (03.06.1985), claims 1 to 4; page 2, lower right column; example 1 (Family: none) | 1-17 |
| Y | CHEN, Y et al, A bilayer prototype woven vascular prosthesis with improved radial compliance, The Journal of The Textile Institute, 2012, Vol.103, No.1, p.106-111 ISSN:0040-5000, Abstract | 1-17 |
| Y | JP 2005-80960 A (Du Pont-Toray Co., Ltd.), 31 March 2005 (31.03.2005), claim 2 (Family: none) | 5 |
| Y | WO 2014/168198 A1 (Toray Industries, Inc.), 16 October 2014 (16.10.2014), claims 1 to 8 & US 2016/0067065 A1 claims 1 to 8 & EP 2985042 A1 & CN 105073148 A | 10-14,17 |
| Y | WO 2014/168197 A1 (Toray Industries, Inc.), 16 October 2014 (16.10.2014), claims 1 to 12 & US 2016/0067066 A1 claims 1 to 12 & EP 2985041 A1 & CN 105102007 A | 10,15-17 |
| A | JP 64-5544 A (Toray Industries, Inc.), 10 January 1989 (10.01.1989), claims 1 to 4 & JP 5-111505 A | 1-17 |

Form PCT/ISA/210 (continuation of second sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2718571 B
- JP 2003329146 A
- JP H0880342 A
- JP 3799626 B
- JP H0548132 B
- JP H0588611 B
- JP S614546 B
- JP S6158190 B
- JP S6352898 B
- JP H0528143 B
- JP 2009545333 A
- JP 4152075 B
- JP 3497612 B
- JP S6041947 B
- JP S6047287 B
- JP 4273965 B
- JP H10151192 A
- JP H0824686 B
- JP H07265338 A
- JP 4461217 B
- WO 08032758 A
- WO 12176861 A
- US 3531368 A
- US 3350488 A


### Non-patent literature cited in the description


- *JIS L,* 2010, 1013
- **P. C. BEGOVAC et al.** *Eur Vasc Endovasc Surg,* 2003, vol. 25, 432-437